# EUROPEAN PATENT SPECIFICATION

(11) **EP 2 910 118 B1**
(45) Date of publication and mention of the grant of the patent: **18.10.2017**
(21) Application number: 14155868.4
(22) Date of filing: 20.02.2014
(51) Int. Cl.: A01K 67/027, C07K 14/705, G01N 33/94

(54) **Mutated receptor polypeptide and use thereof**
Mutierte Rezeptorproteine und deren Verwendung
Polypeptide de récepteur mutant et son utilisation

(43) Date of publication of application: 26.08.2015
(73) Proprietor: Noori, Hamid Reza, 72070 Tübingen (DE)
(72) Inventor: Noori, Hamid Reza, 72070 Tübingen (DE); Urbassek, Herbert, 67705 Trippstadt (DE); Mücksch, Christian, 67655 Kaiserslautern (DE); Spanagel, Rainer, 69126 Heidelberg (DE)
(74) Representative: Jacobi, Markus Alexander

(56) References cited:
- US-A1- 2005 170 360
- Deborah Hartmann: "Coexpression of two distinct muscle acetylcholine receptor alpha-subunits during development", Nature, vol. 343 25 January 1990 (1990-01-25), pages 372-375, XP055123979, DOI: 10.1038/343372a0 Retrieved from the Internet: URL:http://www.nature.com/nature/journal/v 343/n6256/pdf/343372a0.pdf [retrieved on 2014-06-18]
- DATABASE UniProt [Online] 16 April 2014 (2014-04-16), "SubName: Full=Uncharacterized protein; Flags: Fragment;", XP002725977, retrieved from EBI accession no. UNIPROT:W5MKA6 Database accession no. W5MKA6

### SEQUENCE LISTING

<110> Noori, Hamid R.
<120> Mutated Receptor Polypeptide and Use Thereof
<130> HNO69023EP
<160> 23
<170> PatentIn version 3.5
<210> 1
   <211> 461
   <212> PRT
   <213> Torpedo marmorata
<400> 1
   Met Ile Leu Cys Ser Tyr Trp His Val Gly Leu Val Leu Leu Leu Phe
   1               5                   10                  15
   Ser Cys Cys Gly Leu Val Leu Gly Ser Glu His Glu Thr Arg Leu Val
               20                  25                  30
   Ala Asn Leu Leu Glu Asn Tyr Asn Lys Val Ile Arg Pro Val Glu His
           35                  40                  45
   His Thr His Phe Val Asp Ile Thr Val Gly Leu Gln Leu Ile Gln Leu
       50                  55                  60
   Ile Asn Val Asp Glu Val Asn Gln Ile Val Glu Thr Asn Val Arg Leu
   65                  70                  75                  80
   Arg Gln Gln Trp Ile Asp Val Arg Leu Arg Trp Asn Pro Ala Asp Tyr
                   85                  90                  95
   Gly Gly Ile Lys Lys Ile Arg Leu Pro Ser Asp Asp Val Trp Leu Pro
               100                 105                 110
   Asp Leu Val Leu Tyr Asn Asn Ala Asp Gly Asp Phe Ala Ile Val His
           115                 120                 125
   Met Thr Lys Leu Leu Leu Asp Tyr Thr Gly Lys Ile Met Trp Thr Pro
       130                 135                 140
   Pro Ala Ile Phe Lys Ser Tyr Cys Glu Ile Ile Val Thr His Phe Pro
   145                 150                 155                 160
   Phe Asp Gln Gln Asn Cys Thr Met Lys Leu Gly Ile Trp Thr Tyr Asp
                   165                 170                 175
   Gly Thr Lys Val Ser Ile Ser Pro Glu Ser Asp Arg Pro Asp Leu Ser
               180                 185                 190
   Thr Phe Met Glu Ser Gly Glu Trp Val Met Lys Asp Tyr Arg Gly Trp
           195                 200                 205
   Lys His Trp Val Tyr Tyr Thr Cys Cys Pro Asp Thr Pro Tyr Leu Asp
       210                 215                 220
   Ile Thr Tyr His Phe Ile Met Gln Arg Ile Pro Leu Tyr Phe Val Val
   225                 230                 235                 240
   Asn Val Ile Ile Pro Cys Leu Leu Phe Ser Phe Leu Thr Val Leu Val
                   245                 250                 255
   Phe Tyr Leu Pro Thr Asp Ser Gly Glu Lys Met Thr Leu Ser Ile Ser
               260                 265                 270
   Val Leu Leu Ser Leu Thr Val Phe Leu Leu Val Ile Val Glu Leu Ile
           275                 280                 285
   Pro Ser Thr Ser Ser Ala Val Pro Leu Ile Gly Lys Tyr Met Leu Phe
       290                 295                 300
   Thr Met Ile Phe Val Ile Ser Ser Ile Ile Val Thr Val Val Val Ile
   305                 310                 315                 320
   Asn Thr His His Arg Ser Pro Ser Thr His Thr Met Pro Gln Trp Val
                   325                 330                 335
   Arg Lys Ile Phe Ile Asn Thr Ile Pro Asn Leu Met Phe Phe Ser Thr
               340                 345                 350
   Met Lys Arg Ala Ser Lys Glu Lys Gln Glu Asn Lys Ile Phe Ala Asp
           355                 360                 365
   Asp Ile Asp Ile Ser Asp Ile Ser Gly Lys Gln Val Thr Gly Glu Val
       370                 375                 380
   Ile Phe Gln Thr Pro Leu Ile Lys Asn Pro Asp Val Lys Ser Ala Ile
   385                 390                 395                 400
   Glu Gly Val Lys Tyr Ile Ala Glu His Met Lys Ser Asp Glu Glu Ser
                   405                 410                 415
   Ser Asn Ala Ala Glu Glu Trp Lys Tyr Val Ala Met Val Ile Asp His
               420                 425                 430
   Ile Leu Leu Cys Val Phe Met Leu Ile Cys Ile Ile Gly Thr Val Cys
           435                 440                 445
   Val Phe Ala Gly Arg Leu Ile Glu Leu Ser Gln Glu Gly
   450 455 460
<210> 2
   <211> 461
   <212> PRT
   <213> Torpedo californica
<400> 2
   Met Ile Leu Cys Ser Tyr Trp His Val Gly Leu Val Leu Leu Leu Phe
   1               5                   10                  15
   Ser Cys Cys Gly Leu Val Leu Gly Ser Glu His Glu Thr Arg Leu Val
               20                  25                  30
   Ala Asn Leu Leu Glu Asn Tyr Asn Lys Val Ile Arg Pro Val Glu His
           35                  40                  45
   His Thr His Phe Val Asp Ile Thr Val Gly Leu Gln Leu Ile Gln Leu
       50                  55                  60
   Ile Ser Val Asp Glu Val Asn Gln Ile Val Glu Thr Asn Val Arg Leu
   65                  70                  75                  80
   Arg Gln Gln Trp Ile Asp Val Arg Leu Arg Trp Asn Pro Ala Asp Tyr
                   85                  90                  95
   Gly Gly Ile Lys Lys Ile Arg Leu Pro Ser Asp Asp Val Trp Leu Pro
               100                 105                 110
   Asp Leu Val Leu Tyr Asn Asn Ala Asp Gly Asp Phe Ala Ile Val His
           115                 120                 125
   Met Thr Lys Leu Leu Leu Asp Tyr Thr Gly Lys Ile Met Trp Thr Pro
       130                 135                 140
   Pro Ala Ile Phe Lys Ser Tyr Cys Glu Ile Ile Val Thr His Phe Pro
   145                 150                 155                 160
   Phe Asp Gln Gln Asn Cys Thr Met Lys Leu Gly Ile Trp Thr Tyr Asp
                   165                 170                 175
   Gly Thr Lys Val Ser Ile Ser Pro Glu Ser Asp Arg Pro Asp Leu Ser
               180                 185                 190
   Thr Phe Met Glu Ser Gly Glu Trp Val Met Lys Asp Tyr Arg Gly Trp
           195                 200                 205
   Lys His Trp Val Tyr Tyr Thr Cys Cys Pro Asp Thr Pro Tyr Leu Asp
       210                 215                 220
   Ile Thr Tyr His Phe Ile Met Gln Arg Ile Pro Leu Tyr Phe Val Val
   225                 230                 235                 240
   Asn Val Ile Ile Pro Cys Leu Leu Phe Ser Phe Leu Thr Gly Leu Val
                   245                 250                 255
   Phe Tyr Leu Pro Thr Asp Ser Gly Glu Lys Met Thr Leu Ser Ile Ser
               260                 265                 270
   Val Leu Leu Ser Leu Thr Val Phe Leu Leu Val Ile Val Glu Leu Ile
           275                 280                 285
   Pro Ser Thr Ser Ser Ala Val Pro Leu Ile Gly Lys Tyr Met Leu Phe
       290                 295                 300
   Thr Met Ile Phe Val Ile Ser Ser Ile Ile Ile Thr Val Val Val Ile
   305                 310                 315                 320
   Asn Thr His His Arg Ser Pro Ser Thr His Thr Met Pro Gln Trp Val
                   325                 330                 335
   Arg Lys Ile Phe Ile Asp Thr Ile Pro Asn Val Met Phe Phe Ser Thr
               340                 345                 350
   Met Lys Arg Ala Ser Lys Glu Lys Gln Glu Asn Lys Ile Phe Ala Asp
           355                 360                 365
   Asp Ile Asp Ile Ser Asp Ile Ser Gly Lys Gln Val Thr Gly Glu Val
       370                 375                 380
   Ile Phe Gln Thr Pro Leu Ile Lys Asn Pro Asp Val Lys Ser Ala Ile
   385                 390                 395                 400
   Glu Gly Val Lys Tyr Ile Ala Glu His Met Lys Ser Asp Glu Glu Ser
                   405                 410                 415
   Ser Asn Ala Ala Glu Glu Trp Lys Tyr Val Ala Met Val Ile Asp His
               420                 425                 430
   Ile Leu Leu Cys Val Phe Met Leu Ile Cys Ile Ile Gly Thr Val Ser
           435                 440                 445
   Val Phe Ala Gly Arg Leu Ile Glu Leu Ser Gln Glu Gly
       450                 455                 460
<210> 3
   <211> 482
   <212> PRT
   <213> Homo sapiens
<400> 3
   Met Glu Pro Trp Pro Leu Leu Leu Leu Phe Ser Leu Cys Ser Ala Gly
   1               5                   10                  15
   Leu Val Leu Gly Ser Glu His Glu Thr Arg Leu Val Ala Lys Leu Phe
               20                  25                  30
   Lys Asp Tyr Ser Ser Val Val Arg Pro Val Glu Asp His Arg Gln Val
           35                  40                  45
   Val Glu Val Thr Val Gly Leu Gln Leu Ile Gln Leu Ile Asn Val Asp
       50                  55                  60
   Glu Val Asn Gln Ile Val Thr Thr Asn Val Arg Leu Lys Gln Gly Asp
   65                  70                  75                  80
   Met Val Asp Leu Pro Arg Pro Ser Cys Val Thr Leu Gly Val Pro Leu
                   85                  90                  95
   Phe Ser His Leu Gln Asn Glu Gln Trp Val Asp Tyr Asn Leu Lys Trp
               100                 105                 110
   Asn Pro Asp Asp Tyr Gly Gly Val Lys Lys Ile His Ile Pro Ser Glu
           115                 120                 125
   Lys Ile Trp Arg Pro Asp Leu Val Leu Tyr Asn Asn Ala Asp Gly Asp
       130                 135                 140
   Phe Ala Ile Val Lys Phe Thr Lys Val Leu Leu Gln Tyr Thr Gly His
   145                 150                 155                 160
   Ile Thr Trp Thr Pro Pro Ala Ile Phe Lys Ser Tyr Cys Glu Ile Ile
                   165                 170                 175
   Val Thr His Phe Pro Phe Asp Glu Gln Asn Cys Ser Met Lys Leu Gly
               180                 185                 190
   Thr Trp Thr Tyr Asp Gly Ser Val Val Ala Ile Asn Pro Glu Ser Asp
           195                 200                 205
   Gln Pro Asp Leu Ser Asn Phe Met Glu Ser Gly Glu Trp Val Ile Lys
       210                 215                 220
   Glu Ser Arg Gly Trp Lys His Ser Val Thr Tyr Ser Cys Cys Pro Asp
   225                 230                 235                 240
   Thr Pro Tyr Leu Asp Ile Thr Tyr His Phe Val Met Gln Arg Leu Pro
   245 250 255
   Leu Tyr Phe Ile Val Asn Val Ile Ile Pro Cys Leu Leu Phe Ser Phe
   260 265 270
   Leu Thr Gly Leu Val Phe Tyr Leu Pro Thr Asp Ser Gly Glu Lys Met
   275 280 285
   Thr Leu Ser Ile Ser Val Leu Leu Ser Leu Thr Val Phe Leu Leu Val
   290 295 300
   Ile Val Glu Leu Ile Pro Ser Thr Ser Ser Ala Val Pro Leu Ile Gly
   305 310 315 320
   Lys Tyr Met Leu Phe Thr Met Val Phe Val Ile Ala Ser Ile Ile Ile
   325 330 335
   Thr Val Ile Val Ile Asn Thr His His Arg Ser Pro Ser Thr His Val
   340 345 350
   Met Pro Asn Trp Val Arg Lys Val Phe Ile Asp Thr Ile Pro Asn Ile
   355 360 365
   Met Phe Phe Ser Thr Met Lys Arg Pro Ser Arg Glu Lys Gln Asp Lys
   370 375 380
   Lys Ile Phe Thr Glu Asp Ile Asp Ile Ser Asp Ile Ser Gly Lys Pro
   385 390 395 400
   Gly Pro Pro Pro Met Gly Phe His Ser Pro Leu Ile Lys His Pro Glu
   405 410 415
   Val Lys Ser Ala Ile Glu Gly Ile Lys Tyr Ile Ala Glu Thr Met Lys
   420 425 430
   Ser Asp Gln Glu Ser Asn Asn Ala Ala Ala Glu Trp Lys Tyr Val Ala
   435 440 445
   Met Val Met Asp His Ile Leu Leu Gly Val Phe Met Leu Val Cys Ile
   450 455 460
   Ile Gly Thr Leu Ala Val Phe Ala Gly Arg Leu Ile Glu Leu Asn Gln
   465 470 475 480
   Gln Gly
<210> 4
   <211> 627
   <212> PRT
   <213> Homo sapiens
<400> 4
<210> 5
   <211> 478
   <212> PRT
   <213> Homo sapiens
<400> 5
   Met Leu Leu Trp Val Gln Gln Ala Leu Leu Ala Leu Leu Leu Pro Thr
   1               5                   10                  15
   Leu Leu Ala Gln Gly Glu Ala Arg Arg Ser Arg Asn Thr Thr Arg Pro
               20                  25                  30
   Ala Leu Leu Arg Leu Ser Asp Tyr Leu Leu Thr Asn Tyr Arg Lys Gly
           35                  40                  45
   Val Arg Pro Val Arg Asp Trp Arg Lys Pro Thr Thr Val Ser Ile Asp
       50                  55                  60
   Val Ile Val Tyr Ala Ile Leu Asn Val Asp Glu Lys Asn Gln Val Leu
   65                  70                  75                  80
   Thr Thr Tyr Ile Trp Tyr Arg Gln Tyr Trp Thr Asp Glu Phe Leu Gln
                   85                  90                  95
   Trp Asn Pro Glu Asp Phe Asp Asn Ile Thr Lys Leu Ser Ile Pro Thr
   100 105 110
   Asp Ser Ile Trp Val Pro Asp Ile Leu Ile Asn Glu Phe Val Asp Val
   115 120 125
   Gly Lys Ser Pro Asn Ile Pro Tyr Val Tyr Ile Arg His Gln Gly Glu
   130 135 140
   Val Gln Asn Tyr Lys Pro Leu Gln Val Val Thr Ala Cys Ser Leu Asp
   145 150 155 160
   Ile Tyr Asn Phe Pro Phe Asp Val Gln Asn Cys Ser Leu Thr Phe Thr
   165 170 175
   Ser Trp Leu His Thr Ile Gln Asp Ile Asn Ile Ser Leu Trp Arg Leu
   180 185 190
   Pro Glu Lys Val Lys Ser Asp Arg Ser Val Phe Met Asn Gln Gly Glu
   195 200 205
   Trp Glu Leu Leu Gly Val Leu Pro Tyr Phe Arg Glu Phe Ser Met Glu
   210 215 220
   Ser Ser Asn Tyr Tyr Ala Glu Met Lys Phe Tyr Val Val Ile Arg Arg
   225 230 235 240
   Arg Pro Leu Phe Tyr Val Val Ser Leu Leu Leu Pro Ser Ile Phe Leu
   245 250 255
   Met Val Met Asp Ile Val Gly Phe Tyr Leu Pro Pro Asn Ser Gly Glu
   260 265 270
   Arg Val Ser Phe Lys Ile Thr Leu Leu Leu Gly Tyr Ser Val Phe Leu
   275 280 285
   Ile Ile Val Ser Asp Thr Leu Pro Ala Thr Ala Ile Gly Thr Pro Leu
   290 295 300
   Ile Gly Val Tyr Phe Val Val Cys Met Ala Leu Leu Val Ile Ser Leu
   305 310 315 320
   Ala Glu Thr Ile Phe Ile Val Arg Leu Val His Lys Gln Asp Leu Gln
   325 330 335
   Gln Pro Val Pro Ala Trp Leu Arg His Leu Val Leu Glu Arg Ile Ala
   340 345 350
   Trp Leu Leu Cys Leu Arg Glu Gln Ser Thr Ser Gln Arg Pro Pro Ala
           355                 360                 365
   Thr Ser Gln Ala Thr Lys Thr Asp Asp Cys Ser Ala Met Gly Asn His
       370                 375                 380
   Cys Ser His Met Gly Gly Pro Gln Asp Phe Glu Lys Ser Pro Arg Asp
   385                 390                 395                     400
   Arg Cys Ser Pro Pro Pro Pro Pro Arg Glu Ala Ser Leu Ala Val Cys
                   405                 410                 415
   Gly Leu Leu Gln Glu Leu Ser Ser Ile Arg Gln Phe Leu Glu Lys Arg
               420                 425                 430
   Asp Glu Ile Arg Glu Val Ala Arg Asp Trp Leu Arg Val Gly Ser Val
           435                 440                 445
   Leu Asp Lys Leu Leu Phe His Ile Tyr Leu Leu Ala Val Leu Ala Tyr
       450                 455                 460
   Ser Ile Thr Leu Val Met Leu Trp Ser Ile Trp Gln Tyr Ala
   465                 470                 475
<210> 6
   <211> 502
   <212> PRT
   <213> Homo sapiens
<400> 6
<210> 7
   <211> 522
   <212> PRT
   <213> Torpedo californica
<400> 7
   Met Gly Asn Ile His Phe Val Tyr Leu Leu Ile Ser Cys Leu Tyr Tyr
   1               5                   10                  15
   Ser Gly Cys Ser Gly Val Asn Glu Glu Glu Arg Leu Ile Asn Asp Leu
               20                  25                  30
   Leu Ile Val Asn Lys Tyr Asn Lys His Val Arg Pro Val Lys His Asn
           35                  40                  45
   Asn Glu Val Val Asn Ile Ala Leu Ser Leu Thr Leu Ser Asn Leu Ile
       50                  55                  60
   Ser Leu Lys Glu Thr Asp Glu Thr Leu Thr Ser Asn Val Trp Met Asp
   65                  70                  75                  80
   His Ala Trp Tyr Asp His Arg Leu Thr Trp Asn Ala Ser Glu Tyr Ser
                   85                  90                  95
   Asp Ile Ser Ile Leu Arg Leu Pro Pro Glu Leu Val Trp Ile Pro Asp
               100                 105                 110
   Ile Val Leu Gln Asn Asn Asn Asp Gly Gln Tyr His Val Ala Tyr Phe
           115                 120                 125
   Cys Asn Val Leu Val Arg Pro Asn Gly Tyr Val Thr Trp Leu Pro Pro
       130                 135                 140
   Ala Ile Phe Arg Ser Ser Cys Pro Ile Asn Val Leu Tyr Phe Pro Phe
   145                 150                 155                 160
   Asp Trp Gln Asn Cys Ser Leu Lys Phe Thr Ala Leu Asn Tyr Asp Ala
                   165                 170                 175
   Asn Glu Ile Thr Met Asp Leu Met Thr Asp Thr Ile Asp Gly Lys Asp
               180                 185                 190
   Tyr Pro Ile Glu Trp Ile Ile Ile Asp Pro Glu Ala Phe Thr Glu Asn
           195                 200                 205
   Gly Glu Trp Glu Ile Ile His Lys Pro Ala Lys Lys Asn Ile Tyr Pro
       210                 215                 220
   Asp Lys Phe Pro Asn Gly Thr Asn Tyr Gln Asp Val Thr Phe Tyr Leu
   225                 230                 235                 240
   Ile Ile Arg Arg Lys Pro Leu Phe Tyr Val Ile Asn Phe Ile Thr Pro
                   245                 250                 255
   Cys Val Leu Ile Ser Phe Leu Ala Ser Leu Ala Phe Tyr Leu Pro Ala
               260                 265                 270
   Glu Ser Gly Glu Lys Met Ser Thr Ala Ile Ser Val Leu Leu Ala Gln
           275                 280                 285
   Ala Val Phe Leu Leu Leu Thr Ser Gln Arg Leu Pro Glu Thr Ala Leu
       290                 295                 300
   Ala Val Pro Leu Ile Gly Lys Tyr Leu Met Phe Ile Met Ser Leu Val
   305                 310                 315                 320
   Thr Gly Val Ile Val Asn Cys Gly Ile Val Leu Asn Phe His Phe Arg
                   325                 330                 335
   Thr Pro Ser Thr His Val Leu Ser Thr Arg Val Lys Gln Ile Phe Leu
               340                 345                 350
   Glu Lys Leu Pro Arg Ile Leu His Met Ser Arg Ala Asp Glu Ser Glu
           355                 360                 365
   Gln Pro Asp Trp Gln Asn Asp Leu Lys Leu Arg Arg Ser Ser Ser Val
       370                 375                 380
   Gly Tyr Ile Ser Lys Ala Gln Glu Tyr Phe Asn Ile Lys Ser Arg Ser
   385                 390                 395                 400
   Glu Leu Met Phe Glu Lys Gln Ser Glu Arg His Gly Leu Val Pro Arg
                   405                 410                 415
   Val Thr Pro Arg Ile Gly Phe Gly Asn Asn Asn Glu Asn Ile Ala Ala
               420                 425                 430
   Ser Asp Gln Leu His Asp Glu Ile Lys Ser Gly Ile Asp Ser Thr Asn
           435                 440                 445
   Tyr Ile Val Lys Gln Ile Lys Glu Lys Asn Ala Tyr Asp Glu Glu Val
       450                 455                 460
   Gly Asn Trp Asn Leu Val Gly Gln Thr Ile Asp Arg Leu Ser Met Phe
   465                 470                 475                 480
   Ile Ile Thr Pro Val Met Val Leu Gly Thr Ile Phe Ile Phe Val Met
                   485                 490                 495
   Gly Asn Phe Asn His Pro Pro Ala Lys Pro Phe Glu Gly Asp Pro Phe
               500                 505                 510
   Asp Tyr Ser Ser Asp His Pro Arg Cys Ala
           515                 520
<210> 8
   <211> 380
   <212> PRT
   <213> Homo sapiens
<400> 8
   Met Arg Lys Ser Pro Gly Leu Ser Asp Cys Leu Trp Ala Trp Ile Leu
   1               5                   10                  15
   Leu Leu Ser Thr Leu Thr Gly Arg Ser Tyr Gly Gln Pro Ser Leu Gln
               20                  25                  30
   Asp Glu Leu Lys Asp Asn Thr Thr Val Phe Thr Arg Ile Leu Asp Arg
           35                  40                  45
   Leu Leu Asp Gly Tyr Asp Asn Arg Leu Arg Pro Gly Leu Gly Glu Arg
       50                  55                  60
   Val Thr Glu Val Lys Thr Asp Ile Phe Val Thr Ser Phe Gly Pro Val
   65                  70                  75                  80
   Ser Asp His Asp Met Glu Tyr Thr Ile Asp Val Phe Phe Arg Gln Ser
                   85                  90                  95
   Trp Lys Asp Glu Arg Leu Lys Phe Lys Gly Pro Met Thr Val Leu Arg
               100                 105                 110
   Leu Asn Asn Leu Met Ala Ser Lys Ile Arg Thr Pro Asp Thr Phe Phe
           115                 120                 125
   His Asn Gly Lys Lys Ser Val Ala His Asn Met Thr Met Pro Asn Lys
       130                 135                 140
   Leu Leu Arg Ile Thr Glu Asp Gly Thr Leu Leu Tyr Thr Met Arg Leu
   145                 150                 155                 160
   Thr Val Arg Ala Glu Cys Pro Met His Leu Glu Asp Phe Pro Met Asp
                   165                 170                 175
   Ala His Ala Cys Pro Leu Lys Phe Gly Ser Tyr Ala Tyr Thr Arg Ala
               180                 185                 190
   Glu Val Val Tyr Glu Trp Thr Arg Glu Pro Ala Arg Ser Val Val Val
           195                 200                 205
   Ala Glu Asp Gly Ser Arg Leu Asn Gln Tyr Asp Leu Leu Gly Gln Thr
       210                 215                 220
   Val Asp Ser Gly Ile Val Gln Ser Ser Thr Gly Glu Tyr Val Val Met
   225                 230                 235                 240
   Thr Thr His Phe His Leu Lys Arg Lys Ile Gly Tyr Phe Val Ile Gln
                   245                 250                 255
   Thr Tyr Leu Pro Cys Ile Met Thr Val Ile Leu Ser Gln Val Ser Phe
               260                 265                 270
   Trp Leu Asn Glu Ser Val Pro Ala Arg Thr Val Phe Gly Val Thr Thr
           275                 280                 285
   Val Leu Thr Met Thr Thr Leu Ser Ile Ser Ala Arg Asn Ser Leu Pro
       290                 295                 300
   Lys Val Ala Tyr Ala Thr Ala Met Asp Trp Phe Ile Ala Val Cys Tyr
   305                 310                 315                 320
   Ala Phe Val Phe Ser Ala Leu Ile Glu Phe Ala Thr Val Asn Tyr Phe
                   325                 330                 335
   Thr Lys Arg Gly Tyr Ala Trp Asp Gly Lys Ser Val Val Pro Glu Lys
               340                 345                 350
   Pro Lys Lys Val Lys Asp Pro Leu Ile Lys Lys Asn Asn Thr Tyr Ala
           355                 360                 365
   Pro Thr Ala Thr Ser Tyr Thr Pro Asn Leu Ala Arg
       370                 375                 380
<210> 9
   <211> 506
   <212> PRT
   <213> Torpedo californica
<400> 9
   Met Val Leu Thr Leu Leu Leu Ile Ile Cys Leu Ala Leu Glu Val Arg
   1               5                   10                  15
   Ser Glu Asn Glu Glu Gly Arg Leu Ile Glu Lys Leu Leu Gly Asp Tyr
               20                  25                  30
   Asp Lys Arg Ile Ile Pro Ala Lys Thr Leu Asp His Ile Ile Asp Val
           35                  40                  45
   Thr Leu Lys Leu Thr Leu Thr Asn Leu Ile Ser Leu Asn Glu Lys Glu
       50                  55                  60
   Glu Ala Leu Thr Thr Asn Val Trp Ile Glu Ile Gln Trp Asn Asp Tyr
   65                  70                  75                  80
   Arg Leu Ser Trp Asn Thr Ser Glu Tyr Glu Gly Ile Asp Leu Val Arg
                   85                  90                  95
   Ile Pro Ser Glu Leu Leu Trp Leu Pro Asp Val Val Leu Glu Asn Asn
               100                 105                 110
   Val Asp Gly Gln Phe Glu Val Ala Tyr Tyr Ala Asn Val Leu Val Tyr
           115                 120                 125
   Asn Asp Gly Ser Met Tyr Trp Leu Pro Pro Ala Ile Tyr Arg Ser Thr
       130                 135                 140
   Cys Pro Ile Ala Val Thr Tyr Phe Pro Phe Asp Trp Gln Asn Cys Ser
   145                 150                 155                 160
   Leu Val Phe Arg Ser Gln Thr Tyr Asn Ala His Glu Val Asn Leu Gln
                   165                 170                 175
   Leu Ser Ala Glu Glu Gly Glu Ala Val Glu Trp Ile His Ile Asp Pro
               180                 185                 190
   Glu Asp Phe Thr Glu Asn Gly Glu Trp Thr Ile Arg His Arg Pro Ala
           195                 200                 205
   Lys Lys Asn Tyr Asn Trp Gln Leu Thr Lys Asp Asp Thr Asp Phe Gln
       210                 215                 220
   Glu Ile Ile Phe Phe Leu Ile Ile Gln Arg Lys Pro Leu Phe Tyr Ile
   225                 230                 235                 240
   Ile Asn Ile Ile Ala Pro Cys Val Leu Ile Ser Ser Leu Val Val Leu
                   245                 250                 255
   Val Tyr Phe Leu Pro Ala Gln Ala Gly Gly Gln Lys Cys Thr Leu Ser
               260                 265                 270
   Ile Ser Val Leu Leu Ala Gln Thr Ile Phe Leu Phe Leu Ile Ala Gln
           275                 280                 285
   Lys Val Pro Glu Thr Ser Leu Asn Val Pro Leu Ile Gly Lys Tyr Leu
       290                 295                 300
   Ile Phe Val Met Phe Val Ser Met Leu Ile Val Met Asn Cys Val Ile
   305                 310                 315                 320
   Val Leu Asn Val Ser Leu Arg Thr Pro Asn Thr His Ser Leu Ser Glu
                   325                 330                 335
   Lys Ile Lys His Leu Phe Leu Gly Phe Leu Pro Lys Tyr Leu Gly Met
               340                 345                 350
   Gln Leu Glu Pro Ser Glu Glu Thr Pro Glu Lys Pro Gln Pro Arg Arg
           355                 360                 365
   Arg Ser Ser Phe Gly Ile Met Ile Lys Ala Glu Glu Tyr Ile Leu Lys
       370                 375                 380
   Lys Pro Arg Ser Glu Leu Met Phe Glu Glu Gln Lys Asp Arg His Gly
   385                 390                 395                 400
   Leu Lys Arg Val Asn Lys Met Thr Ser Asp Ile Asp Ile Gly Thr Thr
                   405                 410                 415
   Val Asp Leu Tyr Lys Asp Leu Ala Asn Phe Ala Pro Glu Ile Lys Ser
               420                 425                 430
   Cys Val Glu Ala Cys Asn Phe Ile Ala Lys Ser Thr Lys Glu Gln Asn
           435                 440                 445
   Asp Ser Gly Ser Glu Asn Glu Asn Trp Val Leu Ile Gly Lys Val Ile
       450                 455                 460
   Asp Lys Ala Cys Phe Trp Ile Ala Leu Leu Leu Phe Ser Ile Gly Thr
   465                 470                 475                 480
   Leu Ala Ile Phe Leu Thr Gly His Phe Asn Gln Val Pro Glu Phe Pro
                   485                 490                 495
   Phe Pro Gly Asp Pro Arg Lys Tyr Val Pro
               500                 505
<210> 10
   <211> 493
   <212> PRT
   <213> Torpedo californica
<400> 10
   Met Glu Asn Val Arg Arg Met Ala Leu Gly Leu Val Val Met Met Ala
   1               5                   10                  15
   Leu Ala Leu Ser Gly Val Gly Ala Ser Val Met Glu Asp Thr Leu Leu
               20                  25                  30
   Ser Val Leu Phe Glu Thr Tyr Asn Pro Lys Val Arg Pro Ala Gln Thr
           35                  40                  45
   Val Gly Asp Lys Val Thr Val Arg Val Gly Leu Thr Leu Thr Asn Leu
       50                  55                  60
   Leu Ile Leu Asn Glu Lys Ile Glu Glu Met Thr Thr Asn Val Phe Leu
   65                  70                  75                  80
   Asn Leu Ala Trp Thr Asp Tyr Arg Leu Gln Trp Asp Pro Ala Ala Tyr
                   85                  90                  95
   Glu Gly Ile Lys Asp Leu Arg Ile Pro Ser Ser Asp Val Trp Gln Pro
               100                 105                 110
   Asp Ile Val Leu Met Asn Asn Asn Asp Gly Ser Phe Glu Ile Thr Leu
           115                 120                 125
   His Val Asn Val Leu Val Gln His Thr Gly Ala Val Ser Trp Gln Pro
   130 135 140
   Ser Ala Ile Tyr Arg Ser Ser Cys Thr Ile Lys Val Met Tyr Phe Pro
   145 150 155 160
   Phe Asp Trp Gln Asn Cys Thr Met Val Phe Lys Ser Tyr Thr Tyr Asp
   165 170 175
   Thr Ser Glu Val Thr Leu Gln His Ala Leu Asp Ala Lys Gly Glu Arg
   180 185 190
   Glu Val Lys Glu Ile Val Ile Asn Lys Asp Ala Phe Thr Glu Asn Gly
   195 200 205
   Gln Trp Ser Ile Glu His Lys Pro Ser Arg Lys Asn Trp Arg Ser Asp
   210 215 220
   Asp Pro Ser Tyr Glu Asp Val Thr Phe Tyr Leu Ile Ile Gln Arg Lys
   225 230 235 240
   Pro Leu Phe Tyr Ile Val Tyr Thr Ile Ile Pro Cys Ile Leu Ile Ser
   245 250 255
   Ile Leu Ala Ile Leu Val Phe Tyr Leu Pro Pro Asp Ala Gly Glu Lys
   260 265 270
   Met Ser Leu Ser Ile Ser Ala Leu Leu Ala Val Thr Val Phe Leu Leu
   275 280 285
   Leu Leu Ala Asp Lys Val Pro Glu Thr Ser Leu Ser Val Pro Ile Ile
   290 295 300
   Ile Arg Tyr Leu Met Phe Ile Met Ile Leu Val Ala Phe Ser Val Ile
   305 310 315 320
   Leu Ser Val Val Val Leu Asn Leu His His Arg Ser Pro Asn Thr His
   325 330 335
   Thr Met Pro Asn Trp Ile Arg Gln Ile Phe Ile Glu Thr Leu Pro Pro
   340 345 350
   Phe Leu Trp Ile Gln Arg Pro Val Thr Thr Pro Ser Pro Asp Ser Lys
   355 360 365
   Pro Thr Ile Ile Ser Arg Ala Asn Asp Glu Tyr Phe Ile Arg Lys Pro
   370 375 380
   Ala Gly Asp Phe Val Cys Pro Val Asp Asn Ala Arg Val Ala Val Gln
   385 390 395 400
   Pro Glu Arg Leu Phe Ser Glu Met Lys Trp His Leu Asn Gly Leu Thr
   405 410 415
   Gln Pro Val Thr Leu Pro Gln Asp Leu Lys Glu Ala Val Glu Ala Ile
   420 425 430
   Lys Tyr Ile Ala Glu Gln Leu Glu Ser Ala Ser Glu Phe Asp Asp Leu
   435 440 445
   Lys Lys Asp Trp Gln Tyr Val Ala Met Val Ala Asp Arg Leu Phe Leu
   450 455 460
   Tyr Val Phe Phe Val Ile Cys Ser Ile Gly Thr Phe Ser Ile Phe Leu
   465 470 475 480
   Asp Ala Ser His Asn Val Pro Pro Asp Asn Pro Phe Ala
   485 490
<210> 11
   <211> 502
   <212> PRT
   <213> Homo sapiens
<400> 11
<210> 12
   <211> 449
   <212> PRT
   <213> Homo sapiens
<400> 12
<210> 13
   <211> 409
   <212> PRT
   <213> Homo sapiens
<400> 13
   Met Leu Gln Arg Trp Phe Leu Pro Arg Ser Leu Asn Glu Ser Pro Gly
   1               5                   10                  15
   Gln Asn Gln Lys Glu Glu Lys Leu Cys Thr Glu Asn Phe Thr Arg Ile
               20                  25                  30
   Leu Asp Ser Leu Leu Asp Gly Tyr Asp Asn Arg Leu Arg Pro Gly Phe
           35                  40                  45
   Gly Gly Pro Val Thr Glu Val Lys Thr Asp Ile Tyr Val Thr Ser Phe
       50                  55                  60
   Gly Pro Val Ser Asp Val Glu Met Glu Tyr Thr Met Asp Val Phe Phe
   65                  70                  75                  80
   Arg Gln Thr Trp Ile Asp Lys Arg Leu Lys Tyr Asp Gly Pro Ile Glu
                   85                  90                  95
   Ile Leu Arg Leu Asn Asn Met Met Val Thr Lys Val Trp Thr Pro Asp
               100                 105                 110
   Thr Phe Phe Arg Asn Gly Lys Lys Ser Val Ser His Asn Met Thr Ala
           115                 120                 125
   Pro Asn Lys Leu Phe Arg Ile Met Arg Asn Gly Thr Ile Leu Tyr Thr
       130                 135                 140
   Met Arg Leu Thr Ile Ser Ala Glu Cys Pro Met Arg Leu Val Asp Phe
   145                 150                 155                 160
   Pro Met Asp Gly His Ala Cys Pro Leu Lys Phe Gly Ser Tyr Ala Tyr
                   165                 170                 175
   Pro Lys Ser Glu Met Ile Tyr Thr Trp Thr Lys Gly Pro Glu Lys Ser
               180                 185                 190
   Val Glu Val Pro Lys Glu Ser Ser Ser Leu Val Gln Tyr Asp Leu Ile
           195                 200                 205
   Gly Gln Thr Val Ser Ser Glu Thr Ile Lys Ser Ile Thr Gly Ile Thr
       210                 215                 220
   Thr Val Leu Thr Met Thr Thr Leu Ser Ile Ser Ala Arg His Ser Leu
   225                 230                 235                 240
   Pro Lys Val Ser Tyr Ala Thr Ala Met Asp Trp Phe Ile Ala Val Cys
                   245                 250                 255
   Phe Ala Phe Val Phe Ser Ala Leu Ile Glu Phe Ala Ala Val Asn Tyr
               260                 265                 270
   Phe Thr Asn Ile Gln Met Glu Lys Ala Lys Arg Lys Thr Ser Lys Pro
           275                 280                 285
   Pro Gln Glu Val Pro Ala Ala Pro Val Gln Arg Glu Lys His Pro Glu
       290                 295                 300
   Ala Pro Leu Gln Asn Thr Asn Ala Asn Leu Asn Met Arg Lys Arg Thr
   305                 310                 315                 320
   Asn Ala Leu Val His Ser Glu Ser Asp Val Gly Asn Arg Thr Glu Val
                   325                 330                 335
   Gly Asn His Ser Ser Lys Ser Ser Thr Val Val Gln Glu Ser Ser Lys
               340                 345                 350
   Gly Thr Pro Arg Ser Tyr Leu Ala Ser Ser Pro Asn Pro Phe Ser Arg
           355                 360                 365
   Ala Asn Ala Ala Glu Thr Ile Ser Ala Ala Arg Ala Leu Pro Ser Ala
       370                 375                 380
   Ser Pro Thr Ser Ile Arg Thr Gly Tyr Met Pro Arg Lys Ala Ser Val
   385                 390                 395                 400
   Gly Ser Ala Ser Thr Arg His Val Phe
   405
<210> 14
   <211> 411
   <212> PRT
   <213> Homo sapiens
<400> 14
   Met Trp Thr Val Gln Asn Arg Glu Ser Leu Gly Leu Leu Ser Phe Pro
   1               5                   10                  15
   Val Met Ile Thr Met Val Cys Cys Ala His Ser Thr Asn Glu Pro Ser
               20                  25                  30
   Asn Met Ser Tyr Val Lys Glu Thr Val Asp Arg Leu Leu Lys Gly Tyr
           35                  40                  45
   Asp Ile Arg Leu Arg Pro Asp Phe Gly Gly Pro Pro Val Asp Val Gly
       50                  55                  60
   Met Arg Ile Asp Val Ala Ser Ile Asp Met Val Ser Glu Val Asn Met
   65                  70                  75                  80
   Asp Tyr Thr Leu Thr Met Tyr Phe Gln Gln Ser Trp Lys Asp Lys Arg
                   85                  90                  95
   Leu Ser Tyr Ser Gly Ile Pro Leu Asn Leu Thr Leu Asp Asn Arg Val
               100                 105                 110
   Ala Asp Gln Leu Trp Val Pro Asp Thr Tyr Phe Leu Asn Asp Lys Lys
           115                 120                 125
   Ser Phe Val His Gly Val Thr Val Lys Asn Arg Met Ile Arg Leu His
       130                 135                 140
   Pro Asp Gly Thr Val Leu Tyr Gly Leu Arg Ile Thr Thr Thr Ala Ala
   145                 150                 155                 160
   Cys Met Met Asp Leu Arg Arg Tyr Pro Leu Asp Glu Gln Asn Cys Thr
                   165                 170                 175
   Leu Glu Ile Glu Ser Tyr Gly Tyr Thr Thr Asp Asp Ile Glu Phe Tyr
               180                 185                 190
   Trp Asn Gly Gly Glu Gly Ala Val Thr Gly Val Asn Lys Ile Glu Leu
           195                 200                 205
   Pro Gln Phe Ser Ile Val Asp Tyr Lys Met Val Ser Lys Lys Val Glu
           210                 215                 220
   Phe Thr Thr Gly Ala Tyr Pro Arg Leu Ser Leu Ser Phe Arg Leu Lys
   225                 230                 235                 240
   Arg Asn Ile Gly Tyr Phe Ile Leu Gln Thr Tyr Met Pro Ser Thr Leu
                   245                 250                 255
   Ile Thr Ile Leu Ser Trp Val Ser Phe Trp Ile Asn Tyr Asp Ala Ser
               260                 265                 270
   Ala Ala Arg Val Ala Leu Gly Ile Thr Thr Val Leu Thr Met Thr Thr
           275                 280                 285
   Ile Ser Thr His Leu Arg Glu Thr Leu Pro Lys Ile Pro Tyr Val Lys
       290                 295                 300
   Ala Ile Asp Ile Tyr Leu Met Gly Cys Phe Val Phe Val Phe Leu Ala
   305                 310                 315                 320
   Leu Leu Glu Tyr Ala Phe Val Asn Tyr Ile Phe Phe Gly Lys Gly Pro
                   325                 330                 335
   Gln Lys Lys Gly Ala Ser Lys Gln Asp Gln Ser Ala Asn Glu Lys Asn
               340                 345                 350
   Lys Leu Glu Met Asn Lys Val Gln Val Asp Ala His Gly Asn Ile Leu
           355                 360                 365
   Leu Ser Thr Leu Glu Ile Arg Asn Glu Thr Ser Gly Ser Glu Val Leu
       370                 375                 380
   Thr Ser Val Ser Asp Pro Lys Ala Thr Met Tyr Ser Tyr Asp Ser Ala
   385                 390                 395                 400
   Ser Ile Gln Tyr Arg Lys Pro Leu Ser Ser Arg
                   405                 410
<210> 15
   <211> 461
   <212> PRT
   <213> artificial
<220>
   <223> mutated form of alpha 1 polypeptide
<400> 15
   Met Ile Leu Cys Ser Tyr Trp His Val Gly Leu Val Leu Leu Leu Phe
   1               5                   10                  15
   Ser Cys Cys Gly Leu Val Leu Gly Ser Glu His Glu Thr Arg Leu Val
               20                  25                  30
   Ala Asn Leu Leu Glu Asn Tyr Asn Lys Val Ile Arg Pro Val Glu His
           35                  40                  45
   His Thr His Phe Val Asp Ile Thr Val Gly Leu Gln Leu Ile Gln Leu
       50                  55                  60
   Ile Asn Val Asp Glu Val Asn Gln Ile Val Glu Thr Asn Val Arg Leu
   65                  70                  75                  80
   Arg Gln Gln Trp Ile Asp Val Arg Leu Arg Trp Asn Pro Ala Asp Tyr
                   85                  90                  95
   Gly Gly Ile Lys Lys Ile Arg Leu Pro Ser Ser Asp Val Trp Leu Pro
               100                 105                 110
   Asp Leu Val Leu Tyr Asn Asn Ala Asp Gly Asp Phe Ala Ile Val His
           115                 120                 125
   Met Thr Lys Leu Leu Leu Asp Tyr Thr Gly Lys Ile Met Trp Thr Pro
       130                 135                 140
   Pro Ala Ile Phe Lys Ser Tyr Cys Glu Ile Ile Val Thr His Phe Pro
   145                 150                 155                 160
   Phe Asp Gln Gln Asn Cys Thr Met Lys Leu Gly Ile Trp Thr Tyr Asp
                   165                 170                 175
   Gly Thr Lys Val Ser Ile Ser Pro Glu Ser Asp Arg Pro Asp Leu Ser
               180                 185                 190
   Thr Phe Met Glu Ser Gly Glu Trp Val Met Lys Asp Tyr Arg Gly Trp
           195                 200                 205
   Lys His Trp Val Tyr Tyr Thr Cys Cys Pro Asp Thr Pro Tyr Leu Asp
       210                 215                 220
   Ile Thr Tyr His Phe Ile Met Gln Arg Ile Pro Leu Tyr Phe Val Val
   225                 230                 235                 240
   Asn Val Ile Ile Pro Cys Leu Leu Phe Ser Phe Leu Thr Val Leu Val
                   245                 250                 255
   Phe Tyr Leu Pro Thr Asp Ser Gly Glu Lys Met Thr Leu Ser Ile Ser
               260                 265                 270
   Val Leu Leu Ser Leu Thr Val Phe Leu Leu Val Ile Val Glu Leu Ile
   275 280 285
   Pro Ser Thr Ser Ser Ala Val Pro Leu Ile Gly Lys Tyr Met Leu Phe
   290 295 300
   Thr Met Ile Phe Val Ile Ser Ser Ile Ile Val Thr Val Val Val Ile
   305 310 315 320
   Asn Thr His His Arg Ser Pro Ser Thr His Thr Met Pro Gln Trp Val
   325 330 335
   Arg Lys Ile Phe Ile Asn Thr Ile Pro Asn Leu Met Phe Phe Ser Thr
   340 345 350
   Met Lys Arg Ala Ser Lys Glu Lys Gln Glu Asn Lys Ile Phe Ala Asp
   355 360 365
   Asp Ile Asp Ile Ser Asp Ile Ser Gly Lys Gln Val Thr Gly Glu Val
   370 375 380
   Ile Phe Gln Thr Pro Leu Ile Lys Asn Pro Asp Val Lys Ser Ala Ile
   385 390 395 400
   Glu Gly Val Lys Tyr Ile Ala Glu His Met Lys Ser Asp Glu Glu Ser
   405 410 415
   Ser Asn Ala Ala Glu Glu Trp Lys Tyr Val Ala Met Val Ile Asp His
   420 425 430
   Ile Leu Leu Cys Val Phe Met Leu Ile Cys Ile Ile Gly Thr Val Cys
   435 440 445
   Val Phe Ala Gly Arg Leu Ile Glu Leu Ser Gln Glu Gly
   450 455 460
<210> 16
   <211> 461
   <212> PRT
   <213> artificial
<220>
   <223> mutated form of alpha 1 polypeptide
<400> 16
   Met Ile Leu Cys Ser Tyr Trp His Val Gly Leu Val Leu Leu Leu Phe
   1               5                   10                  15
   Ser Cys Cys Gly Leu Val Leu Gly Ser Glu His Glu Thr Arg Leu Val
               20                  25                  30
   Ala Asn Leu Leu Glu Asn Tyr Asn Lys Val Ile Arg Pro Val Glu His
           35                  40                  45
   His Thr His Phe Val Asp Ile Thr Val Gly Leu Gln Leu Ile Gln Leu
       50                  55                  60
   Ile Ser Val Asp Glu Val Asn Gln Ile Val Glu Thr Asn Val Arg Leu
   65                  70                  75                  80
   Arg Gln Gln Trp Ile Asp Val Arg Leu Arg Trp Asn Pro Ala Asp Tyr
                   85                  90                  95
   Gly Gly Ile Lys Lys Ile Arg Leu Pro Ser Ser Asp Val Trp Leu Pro
               100                 105                 110
   Asp Leu Val Leu Tyr Asn Asn Ala Asp Gly Asp Phe Ala Ile Val His
           115                 120                 125
   Met Thr Lys Leu Leu Leu Asp Tyr Thr Gly Lys Ile Met Trp Thr Pro
       130                 135                 140
   Pro Ala Ile Phe Lys Ser Tyr Cys Glu Ile Ile Val Thr His Phe Pro
   145                 150                 155                 160
   Phe Asp Gln Gln Asn Cys Thr Met Lys Leu Gly Ile Trp Thr Tyr Asp
                   165                 170                 175
   Gly Thr Lys Val Ser Ile Ser Pro Glu Ser Asp Arg Pro Asp Leu Ser
               180                 185                 190
   Thr Phe Met Glu Ser Gly Glu Trp Val Met Lys Asp Tyr Arg Gly Trp
           195                 200                 205
   Lys His Trp Val Tyr Tyr Thr Cys Cys Pro Asp Thr Pro Tyr Leu Asp
       210                 215                 220
   Ile Thr Tyr His Phe Ile Met Gln Arg Ile Pro Leu Tyr Phe Val Val
   225                 230                 235                 240
   Asn Val Ile Ile Pro Cys Leu Leu Phe Ser Phe Leu Thr Gly Leu Val
                   245                 250                 255
   Phe Tyr Leu Pro Thr Asp Ser Gly Glu Lys Met Thr Leu Ser Ile Ser
               260                 265                 270
   Val Leu Leu Ser Leu Thr Val Phe Leu Leu Val Ile Val Glu Leu Ile
           275                 280                 285
   Pro Ser Thr Ser Ser Ala Val Pro Leu Ile Gly Lys Tyr Met Leu Phe
                   290                 295                 300
   Thr Met Ile Phe Val Ile Ser Ser Ile Ile Ile Thr Val Val Val Ile
   305                 310                 315                 320
   Asn Thr His His Arg Ser Pro Ser Thr His Thr Met Pro Gln Trp Val
                   325                 330                 335
   Arg Lys Ile Phe Ile Asp Thr Ile Pro Asn Val Met Phe Phe Ser Thr
               340                 345                 350
   Met Lys Arg Ala Ser Lys Glu Lys Gln Glu Asn Lys Ile Phe Ala Asp
           355                 360                 365
   Asp Ile Asp Ile Ser Asp Ile Ser Gly Lys Gln Val Thr Gly Glu Val
       370                 375                 380
   Ile Phe Gln Thr Pro Leu Ile Lys Asn Pro Asp Val Lys Ser Ala Ile
   385                 390                 395                 400
   Glu Gly Val Lys Tyr Ile Ala Glu His Met Lys Ser Asp Glu Glu Ser
                   405                 410                 415
   Ser Asn Ala Ala Glu Glu Trp Lys Tyr Val Ala Met Val Ile Asp His
               420                 425                 430
   Ile Leu Leu Cys Val Phe Met Leu Ile Cys Ile Ile Gly Thr Val Ser
           435                 440                 445
   Val Phe Ala Gly Arg Leu Ile Glu Leu Ser Gln Glu Gly
       450                 455                 460
<210> 17
   <211> 482
   <212> PRT
   <213> artificial
<220>
   <223> mutated form of alpha 1 polyppeptide
<400> 17
   Met Glu Pro Trp Pro Leu Leu Leu Leu Phe Ser Leu Cys Ser Ala Gly
   1               5                   10                  15
   Leu Val Leu Gly Ser Glu His Glu Thr Arg Leu Val Ala Lys Leu Phe
               20                  25                  30
   Lys Asp Tyr Ser Ser Val Val Arg Pro Val Glu Asp His Arg Gln Val
           35                  40                  45
   Val Glu Val Thr Val Gly Leu Gln Leu Ile Gln Leu Ile Asn Val Asp
       50                  55                  60
   Glu Val Asn Gln Ile Val Thr Thr Asn Val Arg Leu Lys Gln Gly Asp
   65                  70                  75                  80
   Met Val Asp Leu Pro Arg Pro Ser Cys Val Thr Leu Gly Val Pro Leu
                   85                  90                  95
   Phe Ser His Leu Gln Asn Glu Gln Trp Val Asp Tyr Asn Leu Lys Trp
               100                 105                 110
   Asn Pro Asp Asp Tyr Gly Gly Val Lys Lys Ile His Ile Pro Ser Ser
           115                 120                 125
   Lys Ile Trp Arg Pro Asp Leu Val Leu Tyr Asn Asn Ala Asp Gly Asp
       130                 135                 140
   Phe Ala Ile Val Lys Phe Thr Lys Val Leu Leu Gln Tyr Thr Gly His
   145                 150                 155                 160
   Ile Thr Trp Thr Pro Pro Ala Ile Phe Lys Ser Tyr Cys Glu Ile Ile
                   165                 170                 175
   Val Thr His Phe Pro Phe Asp Glu Gln Asn Cys Ser Met Lys Leu Gly
               180                 185                 190
   Thr Trp Thr Tyr Asp Gly Ser Val Val Ala Ile Asn Pro Glu Ser Asp
           195                 200                 205
   Gln Pro Asp Leu Ser Asn Phe Met Glu Ser Gly Glu Trp Val Ile Lys
       210                 215                 220
   Glu Ser Arg Gly Trp Lys His Ser Val Thr Tyr Ser Cys Cys Pro Asp
   225                 230                 235                 240
   Thr Pro Tyr Leu Asp Ile Thr Tyr His Phe Val Met Gln Arg Leu Pro
                   245                 250                 255
   Leu Tyr Phe Ile Val Asn Val Ile Ile Pro Cys Leu Leu Phe Ser Phe
               260                 265                 270
   Leu Thr Gly Leu Val Phe Tyr Leu Pro Thr Asp Ser Gly Glu Lys Met
           275                 280                 285
   Thr Leu Ser Ile Ser Val Leu Leu Ser Leu Thr Val Phe Leu Leu Val
       290                 295                 300
   Ile Val Glu Leu Ile Pro Ser Thr Ser Ser Ala Val Pro Leu Ile Gly
   305                 310                 315                 320
   Lys Tyr Met Leu Phe Thr Met Val Phe Val Ile Ala Ser Ile Ile Ile
                   325                 330                 335
   Thr Val Ile Val Ile Asn Thr His His Arg Ser Pro Ser Thr His Val
               340                 345                 350
   Met Pro Asn Trp Val Arg Lys Val Phe Ile Asp Thr Ile Pro Asn Ile
           355                 360                 365
   Met Phe Phe Ser Thr Met Lys Arg Pro Ser Arg Glu Lys Gln Asp Lys
       370                 375                 380
   Lys Ile Phe Thr Glu Asp Ile Asp Ile Ser Asp Ile Ser Gly Lys Pro
   385                 390                 395                 400
   Gly Pro Pro Pro Met Gly Phe His Ser Pro Leu Ile Lys His Pro Glu
                   405                 410                 415
   Val Lys Ser Ala Ile Glu Gly Ile Lys Tyr Ile Ala Glu Thr Met Lys
               420                 425                 430
   Ser Asp Gln Glu Ser Asn Asn Ala Ala Ala Glu Trp Lys Tyr Val Ala
           435                 440                 445
   Met Val Met Asp His Ile Leu Leu Gly Val Phe Met Leu Val Cys Ile
       450                 455                 460
   Ile Gly Thr Leu Ala Val Phe Ala Gly Arg Leu Ile Glu Leu Asn Gln
   465                 470                 475                 480
   Gln Gly
<210> 18
   <211> 627
   <212> PRT
   <213> artificial
<220>
   <223> mutated form of alpha 4 polypeptide
<400> 18
   Met Glu Leu Gly Gly Pro Gly Ala Pro Arg Leu Leu Pro Pro Leu Leu
   1               5                   10                  15
   Leu Leu Leu Gly Thr Gly Leu Leu Arg Ala Ser Ser His Val Glu Thr
               20                  25                  30
   Arg Ala His Ala Glu Glu Arg Leu Leu Lys Lys Leu Phe Ser Gly Tyr
           35                  40                  45
   Asn Lys Trp Ser Arg Pro Val Ala Asn Ile Ser Asp Val Val Leu Val
       50                  55                  60
   Arg Phe Gly Leu Ser Ile Ala Gln Leu Ile Asp Val Asp Glu Lys Asn
   65                  70                  75                  80
   Gln Met Met Thr Thr Asn Val Trp Val Lys Gln Glu Trp His Asp Tyr
                   85                  90                  95
   Lys Leu Arg Trp Asp Pro Ala Asp Tyr Glu Asn Val Thr Ser Ile Arg
               100                 105                 110
   Ile Pro Ser Ser Leu Ile Trp Arg Pro Asp Ile Val Leu Tyr Asn Asn
           115                 120                 125
   Ala Asp Gly Asp Phe Ala Val Thr His Leu Thr Lys Ala His Leu Phe
       130                 135                 140
   His Asp Gly Arg Val Gln Trp Thr Pro Pro Ala Ile Tyr Lys Ser Ser
   145                 150                 155                 160
   Cys Ser Ile Asp Val Thr Phe Phe Pro Phe Asp Gln Gln Asn Cys Thr
                   165                 170                 175
   Met Lys Phe Gly Ser Trp Thr Tyr Asp Lys Ala Lys Ile Asp Leu Val
               180                 185                 190
   Asn Met His Ser Arg Val Asp Gln Leu Asp Phe Trp Glu Ser Gly Glu
           195                 200                 205
   Trp Val Ile Val Asp Ala Val Gly Thr Tyr Asn Thr Arg Lys Tyr Glu
       210                 215                 220
   Cys Cys Ala Glu Ile Tyr Pro Asp Ile Thr Tyr Ala Phe Val Ile Arg
   225                 230                 235                 240
   Arg Leu Pro Leu Phe Tyr Thr Ile Asn Leu Ile Ile Pro Cys Leu Leu
                   245                 250                 255
   Ile Ser Cys Leu Thr Val Leu Val Phe Tyr Leu Pro Ser Glu Cys Gly
               260                 265                 270
   Glu Lys Ile Thr Leu Cys Ile Ser Val Leu Leu Ser Leu Thr Val Phe
           275                 280                 285
   Leu Leu Leu Ile Thr Glu Ile Ile Pro Ser Thr Ser Leu Val Ile Pro
   290 295 300
   Leu Ile Gly Glu Tyr Leu Leu Phe Thr Met Ile Phe Val Thr Leu Ser
   305 310 315 320
   Ile Val Ile Thr Val Phe Val Leu Asn Val His His Arg Ser Pro Arg
   325 330 335
   Thr His Thr Met Pro Thr Trp Val Arg Arg Val Phe Leu Asp Ile Val
   340 345 350
   Pro Arg Leu Leu Leu Met Lys Arg Pro Ser Val Val Lys Asp Asn Cys
   355 360 365
   Arg Arg Leu Ile Glu Ser Met His Lys Met Ala Ser Ala Pro Arg Phe
   370 375 380
   Trp Pro Glu Pro Glu Gly Glu Pro Pro Ala Thr Ser Gly Thr Gln Ser
   385 390 395 400
   Leu His Pro Pro Ser Pro Ser Phe Cys Val Pro Leu Asp Val Pro Ala
   405 410 415
   Glu Pro Gly Pro Ser Cys Lys Ser Pro Ser Asp Gln Leu Pro Pro Gln
   420 425 430
   Gln Pro Leu Glu Ala Glu Lys Ala Ser Pro His Pro Ser Pro Gly Pro
   435 440 445
   Cys Arg Pro Pro His Gly Thr Gln Ala Pro Gly Leu Ala Lys Ala Arg
   450 455 460
   Ser Leu Ser Val Gln His Met Ser Ser Pro Gly Glu Ala Val Glu Gly
   465 470 475 480
   Gly Val Arg Cys Arg Ser Arg Ser Ile Gln Tyr Cys Val Pro Arg Asp
   485 490 495
   Asp Ala Ala Pro Glu Ala Asp Gly Gln Ala Ala Gly Ala Leu Ala Ser
   500 505 510
   Arg Asn Thr His Ser Ala Glu Leu Pro Pro Pro Asp Gln Pro Ser Pro
   515 520 525
   Cys Lys Cys Thr Cys Lys Lys Glu Pro Ser Ser Val Ser Pro Ser Ala
   530 535 540
   Thr Val Lys Thr Arg Ser Thr Lys Ala Pro Pro Pro His Leu Pro Leu
   545 550 555 560
   Ser Pro Ala Leu Thr Arg Ala Val Glu Gly Val Gln Tyr Ile Ala Asp
                   565                 570                 575
   His Leu Lys Ala Glu Asp Thr Asp Phe Ser Val Lys Glu Asp Trp Lys
               580                 585                 590
   Tyr Val Ala Met Val Ile Asp Arg Ile Phe Leu Trp Met Phe Ile Ile
           595                 600                 605
   Val Cys Leu Leu Gly Thr Val Gly Leu Phe Leu Pro Pro Trp Leu Ala
       610                 615                 620
   Gly Met Ile
   625
<210> 19
   <211> 6
   <212> PRT
   <213> artificial
<220>
   <223> point mutated consensus sequence
<220>
   <221> X
   <222> (3)..(3)
   <223> Ser, Ala, Cys or Thr
<220>
   <221> X
   <222> (4)..(4)
   <223> any amino acid moiety
<220>
   <221> x
   <222> (5)..(5)
   <223> Ile or Val
<400> 19
   Pro Ser Xaa Xaa Xaa Trp
   1               5
<210> 20
   <211> 6
   <212> PRT
   <213> artificial
<220>
   <223> ALPHA1_Torpedo_point mutated sequence motif
<400> 20
   Pro Ser Ser Asp Val Trp
   1               5
<210> 21
   <211> 6
   <212> PRT
   <213> artificial
<220>
   <223> ALPHA1_Human_point mutated sequence motif
<400> 21
   Pro Ser Ser Lys Ile Trp
   1               5
<210> 22
   <211> 6
   <212> PRT
   <213> artificial
<220>
   <223> ALPHA4_Human_point mutated sequence motif
<400> 22
   Pro Ser Ser Leu Ile Trp
   1               5
<210> 23
   <211> 6
   <212> PRT
   <213> Homo sapiens
<220>
   <221> X
   <222> (3)..(3)
   <223> Asp or Glu
<220>
   <221> X
   <222> (4)..(4)
   <223> any amino acid moiety
<220>
   <221> X
   <222> (5)..(5)
   <223> Ile or Val
<400> 23
   Pro Ser Xaa Xaa Xaa Trp
   1               5

## Description

The present invention relates to a nicotinic acetylcholine receptor subunit alpha polypeptide comprising a polypeptide motif of the sequence of Pro-Ser-Xaa1-Xaa2-Xaa3-Trp (SEQ ID NO:19), wherein Xaa1 refers to Ser; Xaa2 refers to Lys or Leu; and Xaa3 refers to Ile. Moreover, the invention also relates to a polynucleotide, a cell, which is not a human embryonic stem cell, and a non-human animal comprising such nicotinic acetylcholine receptor subunit alpha polypeptide. The invention further relates to the use of the aforementioned and to methods for determining the effect of the polypeptide according to the present invention *in vitro.*

Nicotinic acetylcholine receptors (nAChRs) are important drug and toxin targets which play a significant role in today's pharmaceutics, pharmacology and toxicology. Hartman and Claudio (1990) Nature 343:372-375 describe the coexpression of two distinct muscle acetylcholine receptor alpha-subunits obtained ifrom Torpedo and Xenopus, respectively, in drug development.

Exemplarily, nAChRs are pharmaceutical targets for muscle relaxant agents such as, e.g., atracurium, and ganglionic blocking agents such as, e.g., hexamethonium. Likewise, nAChRs are also widely known as molecular target structures for numerous natural and artificial toxins such as, e.g, nicotine, α-bungarotoxin and a variety of α-neurotoxines from cobras and mambas.

In general, nAChRs are pentameric receptors, i.e., receptors mainly composed of five subunits forming a pore that is able to influence the membrane potential by enabling sodium influx and potassium efflux upon being triggered by an agonist. The physiological agonist is acetylcholine (ACh). A typical nAChR has a molecular weight of approximately 290 kDa and may be glycosylated.

nAChRs comprise combinations of subunits typically selected from group consisting of subunits of the alpha (α), beta (β), gamma (γ), delta (δ) and epsilon (ε) type, wherein different nAChRs have a different composition of subunits. In particular for alpha and beta subunits each different isotypes are known.

Exemplarily, a nAChR may be an (ααβγδ) nAChR (e.g., ((α1)₂βγδ) nAChR, ((α1)₂β2γδ) nAChR, an ((α1)₂β1δε) nAChR, an ((α4)₂(β2)₃) nAChR, an ((α3)₂(β4)₃) nAChR or an ((α7)₅) nAChR.

In particular those nAChRs comprising an alpha subunit (α) are known to interact with ethanol and play an important role in alcohol dependency and alcohol craving (alcoholism).

Examples for variant neuronal nicotinic alpha receptors are also described by US-A 2005/0170360.

So far, the effect of ethanol on the nAChR activity is however not understood and investigation of the effect of ethanol on cells and non-human animals is hampered by the lack of model systems comprising nAChRs less influenced by ethanol.

Because the presence of a functional nAChR plays an essential role in animal viability and is also important for action potentials in neuronal cell cultures, it is not just possible to knock down or even knock out the expression of nAChR or a subunit thereof as performed for other polypeptides. This would severely harm the animal and significantly influence the neurocellular activity in animals and cells. In many cases, a knock down or even knock out the expression of an nAChR subunit would be fatal to an animal.

Furthermore, also for drug screening and drug design purposes the influence of ethanol on the nAChRs is undesired.

Therefore, there is still an unmet need for nicotinic acetylcholine receptor subunit alpha polypeptides which are less influenced by ethanol and are still functional.

Surprisingly, it has been found that a single point mutation in the sequence of nicotinic acetylcholine receptor subunit alpha polypeptides wherein a negatively charged amino acid moiety is substituted is sufficient for obtaining polypeptides bearing these desired properties, i.e., peptides which are less influenced by ethanol and are still functional.

Disclosed is a nicotinic acetylcholine receptor subunit alpha polypeptide comprising a polypeptide motif of the sequence of
Pro-Ser-Xaa1-Xaa2-Xaa3-Trp (SEQ ID NO:19)
wherein:
Xaa1 refers to Ser, Thr, Ala, Cys or Gly;
Xaa2 refers to any amino acid moiety; and
Xaa3 refers to Ile or Val.

In a first aspect, the present invention relates to a nicotinic acetylcholine receptor subunit alpha polypeptide comprising a polypeptide motif of the sequence of
Pro-Ser-Xaa1-Xaa2-Xaa3-Trp (SEQ ID NO:19)
wherein:
Xaa1 refers to Ser;
Xaa2 refers to Lys or Leu; and
Xaa3 refers to Ile.

As used herein, the nicotinic acetylcholine receptor subunit alpha polypeptide may be any alpha subunit (α) of a nicotinic acetylcholine receptor (nAChR).

Accordingly, when the present invention relates to a polypeptide in the context of the nicotinic acetylcholine receptor subunit alpha, the nicotinic acetylcholine receptor subunit alpha polypeptide (nAChR α-polypeptide) is meant. The nAChR α-polypeptide is also designated as the polypeptide of the present invention.

The person skilled in the art will know that nicotinic acetylcholine receptors (nAChRs) are cholinergic ionotropic receptors that form ligand-gated ion channels in the plasma membranes of various cells. Exemplarily, nAChRs are found on the surface of certain neurons and on the presynaptic and postsynaptic sides of the neuromuscular junction. nAChRs are triggerable by the binding of the neurotransmitter acetylcholine (ACh) and typically also by the xenobiotic nicotine. nAChRs are typically comprise five subunits arranged around a central pore. In a viable cell, the opening of said pore typically enables sodium ion (Na⁺) influx and potassium ion efflux (K⁺) and thereby a change in the membrane potential.

nAChRs are widely found in a large variety of species throughout multicellular animals such as vertebrates and insects. Exemplarily, nAChRs are found in the central nervous system (CNS), the peripheral nervous system and as neuromuscular receptors in the neuromuscular junctions of somatic muscles in vertebrates. Preferably, the nAChRs as used herein are of vertebrate origin, in particular from mammal or fish origin.

As used throughout the present invention, the term "origin" in the context of polypeptides means that the amino acid sequence of a polypeptide is derived from the natural sequence found in the species of origin. Therefore, it will be noted that neither the polypeptide molecule nor the genetic material encoding such polypeptide is necessarily obtained from the respective species of origin directly, but may also be generated in another species heterologously, e.g., by means of heterologous expression. The polypeptide molecule nor the genetic material encoding such polypeptide may be obtained from any biological source or may also be produced synthetically.

The nAChRs subunits arranged around a central pore are polypeptides that, in their natural environment (i.e., in the cellular plasma membrane in complex with the other nAChR subunits) form four transmembrane domains with both the N- and C-terminus located extracellularly.

As used throughout the present invention, the term "polypeptide" may be understood interchangeably with the terms "peptide" and "protein" in the broadest sense as any molecule that is mainly composed of amino acid moieties consecutively linked by amide bonds with another. Herein, the terms "amide bond" and "peptidic bond" may be understood interchangeably. Likewise, also the terms "moiety", "radical" and "residue" may be understood interchangeably. As used herein, the majority amino acid moieties are preferably natural amino acid moieties. More preferably, at least 90%, even more preferably at least 95%, even more preferably at least 97%, even more preferably at least 98%, even more preferably at least 99%, in particular all of the amino acid moieties in the polypeptide are natural amino acid moieties linked via amide bonds with another.

However, the polypeptide may also comprise one or more non-natural, artificial moiety/moieties (e.g., beta amino acid moieties, D-amino acid moieties, alkylated amino acid moieties (e.g., methylated amino acid moieties), aminoxy acid moieties, hydrazine acid moieties, etc.) and/or may comprise one or more further backbone modification(s) (e.g., amide analogues instead of amide bonds (e.g., ketomethylene, vinyl, ketodifluoromethylene, amine, cyclopropene, thioester, sulfinamide, sulphonamide, phosphonamide, hydroxyethylene, E-alkene)) replacing one or more amide bond(s).

The polypeptide of the present invention may comprise one or more linear or branched chain(s) of consecutive amino acid moieties. Preferably the polypeptide of the present invention is a linear chain of consecutive amino acid moieties, herein also designated as "polypeptide strand".

Moreover, the amino acid strand may be modified (e.g., by methylation, alkylation, oxidation, cyclization, dehydration). In particular the polypeptide may or may not be subjected to one or more posttranslational modification(s) and/or be conjugated with one or more non-amino acid moiety/moieties. The termini of the polypeptide may, optionally, be capped by any means known in the art, such as, e.g., amidation, acetylation, methylation, acylation. Posttranslational modifications are well-known in the art and may be but may not be limited to lipidation, phosphorylation, sulfatation, glycosylation, truncation, cyclization of several amino acid moieties, cyclization of the polypeptide strand, oxidation, reduction, decarboxylation, acetylation, amidation, deamidation, disulfide bond formation, pyroglutamate formation, amino acid addition, cofactor addition (e.g., biotinylation, heme addition, eicosanoid addition, steroid addition) and complexation of metal ions, non-metal ions, peptides or small molecules. Moreover, optionally, cellular co-factors, such as the binding of ATP, ADP, NAD⁺, NADH+H⁺, NADP⁺, NADPH+H⁺, metal ions, anions, lipids, etc. may be associated to the polypeptide, irrespective on the biological influence thereof.

In many cases, the polypeptide may be glycosylated at one or more site(s). The polypeptide may or may not be truncated by one, two, three, four, five, six, seven, eight, nine ten or even more amino acid moiety/moieties. Such optional truncation may be found at the N-terminus and/or at the C-terminus. Likewise, the polypeptide may or may not be elongated by one, two, three, four, five, six, seven, eight, nine ten or even more amino acid moiety/moieties. Such optional elongation may be found at the N-terminus and/or at the C-terminus.

A further moiety with which the polypeptide is optionally conjugated with may exemplarily be a labeling moiety. As used in the context of the present invention, the term "labeling moiety" may be understood in the broadest sense as any moiety that can be detected, in particular by a molecular imaging method. Exemplarily, a labeling moiety may be a fluorescence label such as, e.g., a fluorescent polypeptide (e.g., cyan fluorescent protein (CFP), green fluorescent protein (GFP) or yellow fluorescent protein (YFP), red fluorescent protein (RFP), mCherry, etc.), a small-molecule dye (e.g., an Atto dye (e.g., ATTO 390, ATTO 425, ATTO 465, ATTO 488, ATTO 495, ATTO 520, ATTO 532, ATTO 550, ATTO 565, ATTO 590, ATTO 594, ATTO 610, ATTO 611X, ATTO 620, ATTO 633, ATTO 635, ATTO 637, ATTO 647, ATTO 647N, ATTO 655, ATTO 665, ATTO 680, ATTO 700, ATTO 725, ATTO 740), a Cy dye (e.g., Cy3, Cy5, Cy5.5, Cy 7), an Alexa dye (e.g., Alexa Fluor 488, Alexa Fluor 546, Alexa Fluor 647, Alexa Fluor 680, Alexa Fluor 750), a Visen dye (e.g. VivoTag680, VivoTag750), an S dye (e.g., S0387), a DyLight fluorophore (e.g., DyLight 750, DyLight 800), an IRDye (e.g., IRDye 680, IRDye 800), a fluorescein dye (e.g., fluorescein, carboxyfluorescein, fluorescein isothiocyanate (FITC)), a rhodamine dye (e.g., rhodamine, tetramethylrhodamine (TAMRA)) or a HOECHST dye), a quantum dot or a combination of two or more thereof. A fluorescence label may optionally also be conjugated with the polypeptide to enable the easier determination of the polypeptide concentration. When the average number of fluorescent labels per polypeptide molecule and the extinction coefficient of each fluorescent label are known, the concentration may be determined by measuring the absorbance and calculating the concentration by means of the Beer-Lambert Law. Alternatively or additionally, the labeling moiety may be a spin label such as, e.g., ²H or ¹³C suitable for Nuclear Magnetic Resonance (NMR). Alternatively or additionally, the labeling moiety may be radioactive label such as, e.g., ³H, ¹⁴C, ¹²³I, ¹²⁴I, ¹³¹I, ³²P, ^{99m}Tc or lanthanides (e.g., ⁶⁴Gd). In this context, a radioactive label may or may not be suitable for scintillation assays, computer tomography (CT), single-photon emission computed tomography (SPECT) or as a label suitable for Positron Emission Tomography (PET) (e.g., ¹¹C, ¹³N, ¹⁵O, ¹⁸F, ⁸²Rb). Alternatively or additionally, the labeling moiety may be metal atom, metal ion or metal bead (e.g., a gold bead).

As used throughout the present invention, the term "conjugated with" may be understood in the broadest sense as any kind of covalent or non-covalent attachment or linkage of one component with another component, preferably via a covalent linkage. It is not limited to a specific kind of formation of a conjugate. Depending on the components conjugated with another, such conjugate can be obtained by chemical means and/or by genetic engineering and biotechnological means.

Various types of nAChR subunits are known to those skilled in the art such as subunit alpha (α), subunit beta (β), subunit gamma (γ), subunit delta (δ) and subunit epsilon (ε). In particular, with respect to the subunits subunit alpha (α) and beta (β), the person skilled in the art distinguishes between different isotypes such as, e.g., isotypes 1-10 of subunit alpha (α) and isotypes 1-4 of subunit alpha (β). As used herein, the nomenclature as generally used in the art applies.

The nicotinic acetylcholine receptor subunit alpha (nAChR α-polypeptide) according to the present invention may be any nAChR α-polypeptide, therefore, an nAChR α-polypeptide of any species origin and of any isotype. Preferably, the nAChR α-polypeptide of the present invention is of any of isotypes α1-10. More preferably, the nAChR α-polypeptide of the present invention is of any of isotypes α1-6 and 8-10. Even more preferably, the nAChR α-polypeptide of the present invention is of isotype α1 or α4, in particular of isotype α1.

Preferably, the nAChR α-polypeptide is of vertebrate origin. More preferably, the nAChR α-polypeptide is of mammal or fish origin. Even more preferably, the nAChR α-polypeptide is of human (*Homo sapiens*) or ray (e.g., *Torpedo* (e.g., *Torpedo marmorata* or *Torpedo californica*) origin. Particularly preferably, the nAChR α-polypeptide is of human origin, i.e., a human nAChR α-polypeptide.

Exemplarily, when the polypeptide of the present invention is an nAChR α-polypeptide of isotype alpha 1 (α1) from the species origin *Torpedo marmorata,* the respective wild type (wt) form of said polypeptide may have the sequence of SEQ ID NO:1:

When the polypeptide of the present invention is an nAChR α-polypeptide of isotype alpha 1 (α1) from the species origin *Torpedo californica,* the respective wild type (wt) form of said polypeptide may, exemplarily, have the sequence of SEQ ID NO:2:

When the polypeptide of the present invention is a human nAChR α-polypeptide of isotype alpha 1 (α1), the respective wild type (wt) form of said polypeptide may, exemplarily, have the sequence of SEQ ID NO:3:

When the polypeptide of the present invention is a human nAChR α-polypeptide of isotype alpha 4 (α4), the respective wild type (wt) form of said polypeptide may, exemplarily, have the sequence of SEQ ID NO:4:

As used throughout the invention, the respective wild type form is the wild type form of the nicotinic acetylcholine receptor of the same subunit and isoform that the polypeptide according to the present invention it is respective to. Exemplarily, when the polypeptide according to the present invention is a mutated form of the human alpha subunit of nAChR isotype 1, then the respective wild type form is the wild type form of the human alpha subunit of nAChR isotype 1. Herein, the wild type form is preferably that most frequently present throughout population.

Naturally, i.e., in the wild type (wt) form, a wt nAChR α-polypeptide comprises a negatively charged amino acid moiety (Glu or Asp (i.e., a glutamyl moiety or a aspartyl moiety)) in a position located consecutively after a Pro-Ser (prolyl-serinyl) motif and three amino acids before a Trp (tryptophanyl) amino acid moiety. Said negatively charged amino acid moiety is typically located at an amino acid moiety position between 100 and 150 in the amino acid of the wt nAChR α-polypeptide.

As used throughout the present invention, the terms "motif", "polypeptide motif" and "sequence motif" may be understood in the broadest sense as any sequential section of two or more consecutive amino acid moieties in a polypeptide strand linked by amide bonds with another. Preferably, the motif has at least three consecutive amino acid moieties in a polypeptide strand linked by amide bonds with another. More preferably, the motif has at least four consecutive amino acid moieties in a polypeptide strand linked by amide bonds with another. Even more preferably, the motif has at least five consecutive amino acid moieties in a polypeptide strand linked by amide bonds with another. Particularly preferable, the motif has six consecutive amino acid moieties in a polypeptide strand linked by amide bonds with another.

Preferably, the respective wild type (wt) form of a nAChR α-polypeptide comprises the following polypeptide motif:
Pro-Ser-Xaa1-Xaa2-Xaa3-Trp (SEQ ID NO:23)
   wherein:
   Xaa1 refers to Asp or Glu;
   Xaa2 refers to any amino acid moiety, preferably to Lys, Asp or Leu; and
   Xaa3 refers to Ile or Val.

Accordingly, the respective wild type (wt) form of a nAChR α-polypeptide comprises a polypeptide motif containing at least one negatively charged amino acid moiety at a position referring to Xaa1.

In the nAChR α-polypeptide of the present invention, this negatively charged amino acid moiety has been replaced by a neutral small-size amino acid moiety that is not to hydrophobic. Accordingly, said amino acid moiety may be Ser (a serinyl moiety), Thr (a threonyl moiety), Ala (an alanyl moiety) Cys (a cysteinyl moiety) or Gly (a glycyl moiety). Accordingly, the present invention relates to an nAChR α-polypeptide comprising the sequence motif:
Pro-Ser-Xaa1-Xaa2-Xaa3-Trp (SEQ ID NO:19)
   wherein:
   Xaa1 refers to Ser, Thr, Ala, Cys or Gly;
   Xaa2 refers to any amino acid moiety; and
   Xaa3 refers to Ile or Val.

This sequence motif or SEQ ID NO:19 is embedded into the consecutive amino acid polypeptide strand of the nAChR α-polypeptide of the present invention. Herein, it may be located anywhere in the nAChR α-polypeptide.

In a preferred embodiment, the amino acid moiety Xaa1 is located at an amino acid moiety position between 100 and 150 in the amino acid of the nicotinic acetylcholine receptor subunit alpha polypeptide, in particular in a position of between 105 and 130 in the amino acid of the nicotinic acetylcholine receptor subunit alpha polypeptide.

Exemplarily, when the nAChR α-polypeptide of the present invention is a mutated form of the alpha 1 from the species origin *Torpedo marmorata* or *Torpedo californica,* the Xaa1 may preferably be located at an amino acid moiety position between 100 and 115, more preferably at an amino acid moiety position between 105 and 110, in particular at amino acid moiety position of 107.

When the nAChR α-polypeptide of the present invention is, exemplarily, a mutated form of the human alpha 1 subunit, the Xaa1 may preferably be located at an amino acid moiety position between 120 and 140, more preferably at an amino acid moiety position between 125 and 130, in particular at an amino acid moiety position 128.

When the nAChR α-polypeptide of the present invention is, exemplarily, a mutated form of the human alpha 4 subunit, the Xaa1 may preferably be located at an amino acid moiety position between 110 and 130, more preferably at an amino acid moiety position between 115 and 120, in particular at an amino acid moiety position 116.

As defined above, according to the present invention, the Xaa1 is located at position 3 of the polypeptide motif according to SEQ ID NO:19. Xaa1 may be any neutral small-size amino acid moiety that is not to hydrophobic. Therefore, it may exemplarily be Ser, Thr, Ala, Cys or Gly. Preferably, Xaa1 as used herein is Ser, Thr or Ala. More preferably, Xaa1 as used herein is Ser or Thr. Particularly preferably, Xaa1 as used herein is Ser.

Xaa2 of the polypeptide motif according to SEQ ID NO:19 may be any amino acid moiety. Preferably, Xaa2 as used herein is an aliphatic amino acid moiety. More preferably, Xaa2 as used herein is Lys, Asp or Leu. Exemplarily, when the nAChR α-polypeptide of the present invention is a mutated form of the alpha 1 from the species origin *Torpedo marmorata* or *Torpedo californica,* Xaa2 may preferably be Asp. When the nAChR α-polypeptide of the present invention is, exemplarily, a mutated form of the human alpha 1 subunit, Xaa2 may preferably be Lys. When the nAChR α-polypeptide of the present invention is, exemplarily, a mutated form of the human alpha 4 subunit, Xaa2 may preferably be Leu.

Xaa3 of the polypeptide motif according to SEQ ID NO:19 may be Ile or Val. Exemplarily, when the nAChR α-polypeptide of the present invention is a mutated form of the alpha 1 from the species origin *Torpedo marmorata* or *Torpedo californica,* Xaa2 may preferably be Val. When the nAChR α-polypeptide of the present invention is, exemplarily, a mutated form of the human alpha 1 or alpha 4 subunit, Xaa2 may preferably be Ile.

Accordingly, in the polypeptide disclosed herein,
Xaa1 may refer to Ser;
Xaa2 may refer to Lys, Asp or Leu; and
Xaa3 may refer to Ile or Val.

According to the invention, the polypeptide motif is selected from a sequence selected from the group consisting of SEQ ID NO:21 and SEQ ID NO:22. Disclosed is also a polypeptide of SEQ ID NO:20.

Herein, the amino acid sequence of SEQ ID NO:20 refers to
PSSDVW

The amino acid sequence of SEQ ID NO:21 as used herein refers to:
PSSKIW

The amino acid sequence of SEQ ID NO:22 as used herein refers to:
PSSLIW

Disclosed is a mutated form of the acetylcholine receptor alpha subunit isoform 1 of a *Torpedo* species (e.g., *Torpedo marmorata* or *Torpedo californica*), said polypeptide comprises the polypeptide motif having a SEQ ID NO:20.

When the polypeptide according to the present invention is a mutated form of the human acetylcholine receptor alpha subunit isoform 1, said polypeptide comprises the polypeptide motif having a SEQ ID NO:21.

When the polypeptide according to the present invention is a mutated form of the human acetylcholine receptor alpha subunit isoform 4, said polypeptide comprises the polypeptide motif having a SEQ ID NO:22.

Accordingly, in an even more preferred embodiment, the polypeptide is selected from polypeptides having a sequence homology of at least 80% to any one of the sequences of SEQ ID NO:17, SEQ ID NO:18, SEQ ID NO:15 or SEQ ID NO:16.

Herein, the amino acid sequence of SEQ ID NO:15 representing a Mutated form of the acetylcholine receptor alpha subunit isoform 1 from the species origin *Torpedo marmorata* (ALPHA1_TorpedoM_mutated) refers to:

The amino acid sequence of SEQ ID NO:16 representing a Mutated form of the acetylcholine receptor alpha subunit isoform 1 from the species origin *Torpedo californica* (ALPHA1_TorpedoC_mutated) refers to:

The amino acid sequence of SEQ ID NO:17 representing a Mutated form of the acetylcholine receptor alpha subunit isoform 1 from the species origin *Homo sapiens* (ALPHA1_Human_mutated) refers to:

The amino acid sequence of SEQ ID NO:18 representing a Mutated form of the acetylcholine receptor alpha subunit isoform 4 from the species origin *Homo sapiens* (ALPHA4_Human_mutated) refers to:

Also these sequences may optionally be subjected to one or more posttranscriptional modifications. Exemplarily, such posttranscriptional modification may be such as exemplified above. In particular, in many cases, the polypeptide of one of these sequences, in particular when expressed in an eukaryotic cell, may be glycosylated at one or more site(s).

As used herein, the term "sequence homology" may be understood by those skilled in the art. Preferably, sequence homology refers to the standard protein Basic Local Alignment Search Tool (BLAST) as provided by the NCBI in February 2014, more preferably by using standard default (i.e., using a BLOSUM62 matrix; gap costs: existence 11, extension: 1; compositional adjustments: conditional compositional score matrix adjustment; and database non-redundant protein sequences (nr)).

The person skilled in the art will notice that, according to the present invention, every polypeptide in the sense of the present invention having a sequence homology of at least 80% to any one of the sequences of SEQ ID NO:15-18 will also comprise a polypeptide motif according to SEQ ID NO:19.

In particular, a polypeptide of 80% sequence homology may be such having at least 90%, at least 95%, at least 99% or even 100% sequence identity in a strand section of at least 100, at least 200, at least 300, at least 400, at least 500 or even more consecutive amino acid moieties of an amino acid sequence of any of SEQ ID NO:15-18. Then, the sequence may optionally be truncated by one, two, three, four, five, six, seven, eight, nine ten or even more amino acid moiety/moieties. Such optional truncation may be found at the N-terminus and/or at the C-terminus. Likewise, such polypeptide may optionally be elongated by one, two, three, four, five, six, seven, eight, nine ten or even more amino acid moiety/moieties. Such optional elongation may be found at the N-terminus and/or at the C-terminus.

As mentioned above, the polypeptide according to the present invention preferably has a sequence homology of at least 80% to any one of the sequences of SEQ ID NO:17, SEQ ID NO:18, SEQ ID NO:15 or SEQ ID NO:16. More preferably, the polypeptide according to the present invention has a sequence homology of at least 90%, even more preferably at least 95%, even more preferably at least 98%, even more preferably at least 99% to any one of the sequences of SEQ ID NO:17, SEQ ID NO:18, SEQ ID NO:15 or SEQ ID NO:16. Particularly preferably, the polypeptide according to the present invention has a sequence of SEQ ID NO:17 or SEQ ID NO:18. Disclosed are also polypeptides having a sequence ofSEQ ID NO:15 or SEQ ID NO:16.

Furthermore, the polypeptide according to the present invention may preferably also have a structural homology of at least 80% to the respective wild type form of the nAChR α-polypeptide (e.g., SEQ ID NO:17, SEQ ID NO:18, SEQ ID NO:15 or SEQ ID NO:16). Herein, structural homology refers to the homology of the tertiary structure of the polypeptide in its natural environment, i.e., in a membrane-embedded complex in a pentameric nAChR. More preferably, the polypeptide according to the present invention have a structural homology of at least 90%, even more preferably at least 95%, even more preferably at least 98%, even more preferably at least 99% to the respective wild type form of the nAChR α-polypeptide (e.g., SEQ ID NO:17, SEQ ID NO:18, SEQ ID NO:15 or SEQ ID NO:16). Particularly preferably, the polypeptide according to the present invention has essentially the same tertiary structure as the respective wild type form of the nAChR α-polypeptide (e.g., SEQ ID NO:17, SEQ ID NO:18, SEQ ID NO:15 or SEQ ID NO:16).

Accordingly, in an even more preferred embodiment, the polypeptide is selected from polypeptides having a sequence homology of at least 80% to any one of the sequences of SEQ ID NO:17, SEQ ID NO:18, SEQ ID NO:15 or SEQ ID NO:16.

The polypeptide according to the present invention may optionally be dissolved in a buffer, may be frozen (e.g., in water, in a buffer or in a freezing medium comprising additives maintain its functionality) or freeze dried.

Optionally, the nicotinic acetylcholine receptor subunit alpha (nAChR α-polypeptide) of the present invention may be included in a pentameric nicotinic acetylcholine receptor (nAChR). Then, the nAChR may comprise one, two, three, or four nAChR α-polypeptide(s) of the present invention or may even be composed of five nAChR α-polypeptides.

Therefore, the present invention also relates to a nicotinic acetylcholine receptor (nAChR) comprising at least one polypeptide of the present invention.

Preferably, the nAChR comprising at least one polypeptide of the present invention as alpha subunit(s) has Na⁺/K⁺ ion transport rate of at least 25% compared to the respective nAChR comprising wild type subunits only, when measured at comparable conditions (i.e., in a comparable environment in a membrane suitable for its activity, in the presence of an activator such as, e.g., acetylcholine (ACh) or nicotine). More preferably, the nAChR comprising at least one polypeptide of the present invention as alpha subunit(s) has Na⁺/K⁺ ion transport rate of at least 50%, even more preferably of at least 80%, even more preferably of at least 90%, even more preferably of at least 95% compared to the respective nAChR comprising wild type subunits only, when measured at comparable conditions. Particularly preferably the nAChR comprising at least one polypeptide of the present invention as alpha subunit(s) has Na⁺/K⁺ ion transport rate comparable to that of the respective nAChR comprising wild type subunits only, when measured at comparable conditions.

Preferably, such pentameric nAChR may be an (ααβγδ) nAChR. More preferably, such pentameric nAChR may be an ((α1)₂βγδ) nAChR, such as, e.g., an ((α1)₂β2γδ) nAChR composed of two α1, and one β2, γ and δ each, typically found in various muscle cells. Alternatively, it may also be an ((α1)₂β1δε) nAChR also typically found in various muscle cells, an ((α4)₂(β2)₃) nAChR, or ((α7)₅) nAChR typically found in cells of the CNS (e.g., various neuronal cells in the brain) or an ((α3)₂(β4)₃) nAChR typically found in various ganglial cells.

The person skilled in the art will notice that, in this context, the species origin of the other subunits of such nAChR (i.e., other α, β, γ, δ and ε subunits, respectively) are preferably of the same sequence origin as the polypeptide according to the present invention (i.e., the nAChR α-polypeptide according to the present invention). Exemplarily, all subunits comprised in the pentameric nAChR may be of a human origin.

The polypeptide according to the present invention may be obtained by any means. Exemplarily, it may be obtained by genetic engineering and biotechnological means and/or by synthetical means. Preferably, the polypeptide according to the present invention may be obtained by genetic engineering and biotechnological means. Genetic engineering and biotechnological means are based on the expression of the genetic information encoded by a polynucleotide strand.

Therefore, in a further aspect, the present invention also relates to a polynucleotide encoding for a polypeptide according to the present invention.

A polynucleotide as used herein may be any polynucleotide suitable for encoding genetic information expressible in a cell. Exemplarily, a polynucleotide may be a deoxyribonucleic acid (DNA) or a ribonucleic acid (RNA) strand. Alternatively, a polynucleotide may also comprise one or more nucleotide analoga or even consist of nucleotide analoga. Preferably, the polynucleotide according to the present invention is a DNA or an RNA strand.

Particularly preferably, the polynucleotide according to the present invention is a DNA strand. The polynucleotide may be comprised in a linear strand or may be comprised in a circular strand or in a genome (e.g., a eukaryotic genome, a bacterial genome or a viral genome).

It will be noted that the sequence of the polynucleotide according to the present invention may be derived from the sequence of the polypeptide according to the present invention without any burden. In this context, the person skilled in the art may apply the three letter genetic code well-known to anyone skilled in the art.

The polynucleotide according to the present invention may be derived from a natural origin or may be synthetically produced. Preferably, the polynucleotide according to the present invention may be derived from a natural origin. It will be noted that then at least one point mutation will preferably be included into the sequence in order to obtain a polynucleotide wherein a fraction encodes for the polypeptide motif according to SEQ ID NO:19.

This point mutation may be included into the sequence by any means such as any method known for site directed mutagenesis such as, e.g., by means of polymerase chain reaction (PCR) site directed mutagenesis, cassette mutagenesis, Kunkel's method, whole plasmid mutagenesis or even an *in vivo* site directed mutagenesis method. These methods are well-known to those skilled in the art.

Optionally, the polynucleotide according to the present invention may be amplified by any means known in the art such as, e.g., PCR and/or molecular cloning into cells (e.g., bacteria) and propagating said cells. These methods are well-known to those skilled in the art.

The polynucleotide may be isolated or may be embedded into a vector.

In a preferred embodiment, polynucleotide is comprised in a vector.

A vector may be any vector known in the art. Exemplarily, a vector may be a bacterial circular vector (plasmid suitable for bacteria), may be an eukaryotic circular vector (plasmid suitable for eukaryotic cells), may be an eukaryotic or bacterial linear vector, or may be a viral vector, i.e., a virus which viral genome comprises the polynucleotide sequence according to the present invention (e.g., an herpes simplex virus (HSV) or a baculovirus).

The polynucleotide may be expressed to obtain the polypeptide according to the present invention.

Then, the polynucleotide may preferably be embedded into a polynucleotide strand bearing an expression promoter binding site upstream to the polypeptide-encoding sequence (i.e., the polynucleotide sequence according to the present invention) and at least one stop codon at the end of the polypeptide-encoding sequence. Optionally, it may further comprise one or more amplifier sequences. The person skilled in the art will know numerous of said promoter and amplifier sequences which may be preferably chosen to be suitable for the expression system used. In particular, when the vector is a plasmid, said vector may comprise an expression promoter binding site upstream to the polypeptide-encoding sequence and a stop codon downstream of the polypeptide-encoding sequence, and, optionally, one or more enhancer sequences.

The polynucleotide according to the present invention may optionally be dissolved in a buffer, may be frozen (e.g., in water, in a buffer or in a freezing medium comprising additives maintain its functionality), dried or freeze dried.

Expression may be performed in a cell or in a cell-free expression system. Preferably, expression is performed in a cell.

Accordingly, in a still further aspect, the present invention relates to a cell comprising a polypeptide and/or a polynucleotide according to the present invention.

This cell may be any cell except a human embryonic stem cell. Exemplarily, the cell may be a eukaryotic cell (e.g., an animal cell except a human embryonic stem cell (e.g., a mammal or an insect cell) or a fungal cell (e.g., a yeast cell)) or may be a bacterial cell. The cell may be of the species where the polypeptide according to the present invention is derived from (homologous expression). Alternatively, the cell may be of another species (heterologous expression). When the cell is of another species, the cell may typically also be designated as a genetically modified cell. Preferably, the cell is no human embryonic stem cell. When the cell is a cell of animal origin, it is preferably but not necessarily of neuronal origin.

Preferably, the cell expresses also the other subunits present in the nAChR of interests. More preferably, the cell comprises at its cellular membrane nAChR comprising the polypeptide according to the present invention. Accordingly, highly preferably, in the cell, the polypeptide according to the present invention is comprised in an nAChR in an environment maintaining its functionality.

The cell may be optionally transfected with one or more polynucleotide(s) according to the present invention, in particular wherein the polynucleotide(s) is/are embedded into a plasmid. Then, the polypeptide according to the present invention may be expressed and, optionally, overexpressed. Preferably, then, the gene encoding for the respective wild type form of the nAChR α-polypeptide may be knocked out by a partial or complete deletion of the gene or may be altered in a way that it is not functional any longer. Alternatively, the promoter, the start codon or the enhancer(s) may be rendered inactive.

Alternatively, the respective wild type form of the nAChR α-polypeptide may be point mutated (e.g., by site directed mutagenesis) to directly form the polypeptide according to the present invention.

Alternatively, the respective wild type gene may maintained in its natural form, but the expression is hampered, i.e., down-regulated. This may, exemplarily, be achieved by means of interfering RNA (RNAi) such as, e.g., small interfering RNA (siRNA), microRNA (miRNA) or small nuclear RNA (snRNA).

Optionally, the cell may be of human origin, in particular may be a cell line derived from human origin. Exemplarily, the cell may be a HeLa cell, a Hek cell, a Jurkat cell, etc.

The cell may be cultured under standard conditions (e.g., in a suitable culture medium at 37°C) or may be cultured at altered conditions.

In particular when the cell is of the species where the polypeptide according to the present invention is derived from (homogenic expression), it will naturally also express the respective wild type sequence of the nAChR α-polypeptide comprising an Asp or Glu moiety in a position corresponding to Xaa1 of the polypeptide motif according to SEQ ID NO:19. Preferably, however, the cell is essentially free of such wild type nAChR α-polypeptide of the isotype that is mutated.

Accordingly, in a preferred embodiment, the cell is essentially free of the wild type nicotinic acetylcholine receptor polypeptide corresponding to the polypeptide of the present invention present in the cells.

Therefore, when the polypeptide of the present invention is a mutated form of the nAChR α-polypeptide of isotype alpha 1 (α1), the cell may preferably be essentially free of wild type forms of nAChR α-polypeptide of isotype alpha 1 (α1). When the polypeptide of the present invention is a mutated form of the nAChR α-polypeptide of isotype alpha 4 (α4), the cell may preferably be essentially free of wild type forms of nAChR α-polypeptide of isotype alpha 4 (α4).

As used throughout the invention, the term "essentially free" may be understood as a concentration of not more than 10% of the naturally occurring concentration, more preferably not more than 5% of the naturally occurring concentration, even more preferably not more than 2% of the naturally occurring concentration, even more preferably not more than 1% of the naturally occurring concentration, even more preferably not more than 0.5% of the naturally occurring concentration, in particular not more than 0.1% of the naturally occurring concentration.

Exemplarily, a *Torpedo* cell may preferably be free of a polypeptide of SEQ ID NO:1 and 2 when the polypeptide is a nAChR α-polypeptide comprising a motif of SEQ ID NO:20. In particular, a *Torpedo* cell may be free of a polypeptide of SEQ ID NO:1 and 2, when the polypeptide has a sequence of SEQ ID NO:15 or 16.

A human cell may preferably be free of a polypeptide of SEQ ID NO:3 when the polypeptide according to the present invention is a nAChR α-polypeptide comprising a motif of SEQ ID NO:21. In particular, a human cell may be free of a polypeptide of SEQ ID NO:3, when the polypeptide according to the present invention has a sequence of SEQ ID NO:17.

A human cell may preferably be free of a polypeptide of SEQ ID NO:4 when the polypeptide according to the present invention is a nAChR α-polypeptide comprising a motif of SEQ ID NO:22. In particular, a human cell may be free of a polypeptide of SEQ ID NO:4, when the polypeptide according to the present invention has a sequence of SEQ ID NO:18.

A human cell may preferably be free of polypeptides of SEQ ID NOs:3 and 4 when the polypeptides according to the present invention are nAChR α-polypeptides comprising motifs of SEQ ID NOs:21 and 22. In particular, a human cell may be free of polypeptides of SEQ ID NOs:3 and 4, when the polypeptides according to the present invention have sequences of SEQ ID NOs:17 and 18.

The cell according to the present invention may be a cell cultivated in a cell culture, a cell cultivated in a tissue culture or a cell *in vivo,* i.e., in a non-human animal.

Accordingly, a yet further aspect of the present invention relates to a non-human animal comprising cells according to the present invention.

In this context, the non-human animal may be any multicellular non-human animal. Exemplarily, the non-human animal may be a non-human vertebrate or an insect. Preferably, the non-human animal is a non-human vertebrate. More preferably, the non-human animal is a mammal such as, e.g., a mouse, a rat, a hamster or a rabbit. The non-human animal may also be designated as model organism.

The non-human animal may only comprise few of such cells according to the present invention or may comprise many of such cells.

In a preferred embodiment, in at least one tissue essentially all cells of at least one cell type are cells according to the present invention.

This means that, then, the cells of the cell type show a particular reaction pattern arising from the reaction pattern of the polypeptide according to the present invention. This may enable to obtain experimental results that are macroscopically measurable in the non-human animal. Exemplarily, the non-human animal may show altered neurotransmitter concentrations and/or altered behavior. Exemplarily, the non-human animal may have less tendency to ethanol craving and/or altered motoneuronal activity and movement pattern.

The non-human animal may also be a genetically modified non-human organism (GMO). Herein, optionally, all cells of the non-human animal may be genetically modified in a way that they do essentially not express any functional form of the corresponding wild type polypeptide any longer. This may optionally be achieved in the germ cells before the non-human animal is formed or may be achieved by means of viruses at a later stage such as in the adjuvant or adult non-human animal. The germ cell is preferably no human embryonic stem cell. Alternatively, the expression level of the respective wild type polypeptide(s) may also be lowered by means of down-regulating the expression of the expression of said polypeptide.

In an even more preferred embodiment, in the at least one tissue essentially all cells of at least one cell type are cells essentially free of the wild type nicotinic acetylcholine receptor polypeptide corresponding to the polypeptide of the present invention.

This will typically enable to obtain experimental results that are suitably macroscopically measurable in the non-human animal. Exemplarily, the non-human animal may show even more clearly altered neurotransmitter concentrations and/or altered behavior.

Most preferably, essentially all cells in the non-human animal are essentially free of the wild type nicotinic acetylcholine receptor polypeptide corresponding to the polypeptide of the present invention.

The person skilled in the art will notice that a polypeptide, a nicotinic acetylcholine receptor comprising said polypeptide, a polynucleotide, a cell and a non-human animal according to the present invention may be used for various uses.

A still further aspect of the present invention relates to the use of a polypeptide according to the present invention, a nicotinic acetylcholine receptor comprising a polypeptide according to the present invention, a polynucleotide according to the present invention, a cell, which is not a human embryonic stem cell, according to the present invention for determining the effect of a compound or a combination of compounds on the activity of a nicotinic acetylcholine receptor comprising said polypeptide.

Then, the nAChR comprising such polypeptide, the polynucleotide, or the cell (or the non-human animal) may be contacted with one or more compound(s) of interest and the activity of the nAChR may be determined. In a non-human animal, contacting the nAChR with the one or more compound(s) typically includes administering the one or more compound(s) to said non-human animal, either locally or systemically.

A compound of interest as used herein may be any compound that could potentially have an influence on the nAChR comprising the polypeptide of the present invention. Exemplarily, the compound of interest may be a (potential) agonist of the nAChR. Alternatively, the compound of interest may be a (potential) antagonist of the nAChR. Yet alternatively, the compound of interest is assumed to be neither an agonist nor an antagonist of the nAChR but is tested to confirm this assumption.

Also two or more compounds of interest that (potentially) are agonists of the nAChR may be tested to determine if there are synergistic affects. Likewise, also two or more compounds of interest that (potentially) are antagonists of the nAChR may be tested to determine if there are synergistic affects. Alternative also one or more compound(s) of interest that (potentially) is/are agonist(s) of the nAChR may be combined with one or more compound(s) of interest that (potentially) is/are antagonist(s) of the nAChR to determine the interaction.

The one or more compound(s) of interest may also be combined with one or more compound(s) influencing the ACh concentration and/or the intracellular signal transduction indirectly.

When using the nicotinic acetylcholine receptor comprising a polypeptide according to the present invention or the cell (or a non-human animal), exemplarily, the sodium influx and/or the potassium efflux may be measured. In a cell (or a non-human animal), the activity may also be determined by determining the action potential between the extracellular and the intracellular side of a cellular membrane. With respect to a non-human animal, the activity may also be determined systemically in the whole non-human animal, such as, e.g., by determining systemic parameters, neurotransmitter concentrations (local or systemically) and/or alterations in the non-human animal behavior (e.g, altered movement pattern, altered tendency to ethanol craving, reduction of withdrawal symptoms).

When determining the effect of a compound or a combination of compounds on the activity, the activity of the polypeptide, the comprising said polypeptide, the polynucleotide, the cell or the non-human animal, the activity observed for the activity of the polypeptide, the comprising said polypeptide, the polynucleotide, the cell or the non-human animal without the presence of the compound(s) is compared with the respective activity of the polypeptide, the comprising said polypeptide, the polynucleotide, the cell or the non-human animal contacted with the compound(s).

The use for determining the effect of a compound or a combination of compounds on the activity of a nicotinic acetylcholine receptor comprising said polypeptide may be a screening procedure. Exemplarily, the screening procedure may be such for testing a compound library for identifying novel lead compounds, may be such for lead compound optimization wherein a variety of modifications of a lead compound are evaluated (also designated as drug design) or may be such for determining the binding and/or activity pattern of a combination of two or more compounds. Herein, one of the compounds may, exemplarily, be ethanol.

Alternatively, the use for determining the effect of a compound or a combination of compounds on the activity of a nicotinic acetylcholine receptor comprising said polypeptide may be determining the binding and/or activity pattern of one or more compound(s). The compound may be ethanol or may be any pharmaceutically active compound.

The activity of the nicotinic acetylcholine receptor may be determined by any means. Exemplarily, the activity of the nicotinic acetylcholine receptor may be determined experimentally or by computer-assisted simulations.

In a preferred embodiment, the activity of the nicotinic acetylcholine receptor is the rate of ions passing the ion channel of said receptor.

These ions are preferably sodium and/or potassium ions.

As mentioned above, a particular benefit of the present invention is that the activity of a compound may also be determined in the absence of ethanol.

Therefore, in a preferred embodiment, the activity of the nicotinic acetylcholine receptor is determined in the absence of ethanol.

A yet further aspect of the present invention relates to an *in vitro* method for determining the effect of at least one compound on the activity of a nicotinic acetylcholine receptor comprising the polypeptide of the present invention, said method comprising the steps:
(i) providing a polypeptide according to the present invention comprised in a nicotinic acetylcholine receptor in an *in vitro* environment maintaining its functionality;
(ii) contacting said nicotinic acetylcholine receptor comprising said polypeptide of step (i) with said at least one compound in an *in vitro* environment maintaining functionality of said polypeptide;
(iii) determining the activity of said acetylcholine receptor comprising said polypeptide contacted with said at least one compound of step (ii); and
(iv) determining the effect of said at least one compound by means of comparing the activity determined according to step (iii) with the activity determined for the acetylcholine receptor comprising said polypeptide of in a comparable *in vitro* environment without being contacted with said at least one compound.

The method as used herein preferably is a screening procedure as described above. Likewise the activity of a nicotinic acetylcholine receptor is preferably determined as described above.

An *in vitro* environment maintaining the polypeptide's functionality may be any *in vitro* environment suitable for this purpose. Typically, one or more polypeptide(s) according to the present invention is/are hereby complexed in a pentameric nAChR.

In a preferred embodiment, the *in vitro* environment maintaining the functionality of the acetylcholine receptor comprising the polypeptide is a membrane composed of amphiphilic molecules.

Such amphiphilic molecules are preferably a cellular membrane or a membrane-mimicking *in vitro* environment (e.g., a liposome mainly composed of phospholipids or a polymersome).

In a preferred embodiment, the *in vitro* environment maintaining the functionality of the acetylcholine receptor comprising the polypeptide is a cellular membrane.

Such cellular membrane may be the membrane of a viable cell or may be obtained from a cell (e.g., by means of homogenization of cells or red blood cells). More preferably, the cellular membrane is present on a viable cell.

In an even more preferred embodiment, the *in vitro* environment maintaining the functionality of the acetylcholine receptor comprising the polypeptide is a cellular membrane of a viable cell according to the present invention.

As mentioned in the context of the use above, determining the activity of the nicotinic acetylcholine receptor may be performed by any means. Examples are mentioned above what also applies in this context.

In a preferred embodiment, the step of determining the activity of the nicotinic acetylcholine receptor comprising the polypeptide (step iii) is determining the rate of ions passing the ion channel of said receptor.

Herein, as mentioned above, the rate of ions passing the ion channel of the receptor is preferably the rate of sodium influx and/or the rate of potassium efflux.

As mentioned above, a particular benefit of the present invention is that the activity of a compound may also be determined in the absence of ethanol.

Accordingly, in a preferred embodiment, the method is conducted in the absence of ethanol.

As mentioned above, the cell and the non-human animal according to the present invention may also be used to determine the importance and effect of one or more particular nicotinic acetylcholine receptor(s) on the pharmacological effect of ethanol.

Disclosed is further a method for determining the effect of ethanol on a cell or a non-human animal mediated by one or more nicotinic acetylcholine receptor(s) comprising one or more nicotinic acetylcholine receptor subunit alpha polypeptide(s) of interest, comprising the steps:
(i) providing a cell according to the present invention comprising one or more nAChR α-polypeptide(s), wherein said cell is essentially free of the respective wild type nAChR α-polypeptide(s) or a non-human animal according to the present invention, wherein essentially all cells comprised in at least one tissue are such cells;
(ii) contacting said cell or said non-human animal with ethanol;
(iii) determining the effect of the ethanol on said cell or non-human animal according to step (ii); and
(iv) comparing the effect of the ethanol on said cell or non-human animal according to step (iii) with
   (a) a cell only comprising the respective wild type nicotinic acetylcholine receptor(s) or a non-human animal comprising said cells; and/or
   (b) a cell or a non-human animal according to step (i) not contacted with ethanol.

A further aspect of the present invention relates to an *in vitro* method for determining the effect of ethanol on a cell mediated by one or more nicotinic acetylcholine receptor(s) comprising one or more nicotinic acetylcholine receptor subunit alpha polypeptide(s) of interest, comprising the steps:
(i) providing a cell according to the present invention;
(ii) contacting said cell with ethanol;
(iii) determining the effect of the ethanol on said cell according to step (ii); and
(iv) comparing the effect of the ethanol on said cell according to step (iii) with
   (a) a cell, only comprising the respective wild type nicotinic acetylcholine receptor(s); and/or
   (b) a cell according to step (i), not contacted with ethanol.

The nicotinic acetylcholine receptor subunit alpha polypeptide(s) of interest may preferably be the one or more polypeptide(s) of the present invention as defined above and/or the respective wild type form of said polypeptides as defined above.

Herein, the step of contacting said cell (or said non-human animal) with ethanol may be performed by any means. In a non-human animal, the ethanol may be administered systemically or locally such as, e.g., to a particular tissue of interest.

In this context, preferably, the nicotinic acetylcholine receptor subunit alpha polypeptide(s) of interest may be other nicotinic acetylcholine receptor subunit alpha polypeptide(s) than that/those according to the present invention. Alternatively, the nicotinic acetylcholine receptor subunit alpha polypeptide(s) of interest may be nicotinic acetylcholine receptor subunit alpha polypeptide(s) according to the present invention of another or the same isotype.

The effect of the ethanol may be any effect that may be determined by measuring physical and/or (bio)chemical means or by observing the microscopic and/or macroscopic behavior of the cell or non-human animal. Exemplarily, alterations in the sodium influx or potassium efflux may be determined. Additionally or alternatively, alterations in the action potential may be determined. Additionally or alternatively, alterations in the cellular or tissue morphology may be determined. Additionally or alternatively, alterations in the movement pattern of a non-human animal may be determined. Additionally or alternatively, alterations in the alcohol craving of a non-human animal may be determined. Additionally or alternatively, alterations in the reduction of alcohol withdrawal in a non-human animal may be determined (e.g., by observing tremors).

A comparison between the effect of the ethanol on said cell (or non-human animal) according to step (iii) with a cell only comprising the respective wild type nicotinic acetylcholine receptor(s) or a non-human animal comprising said cells may show the specific effect of the ethanol mediated by the nicotinic acetylcholine receptor(s) comprising one or more nicotinic acetylcholine receptor subunit alpha polypeptide(s) of interest on the cell or non-human animal.

A comparison between the effect of the ethanol on said cell (or non-human animal) according to step (iii) with a cell or a non-human animal according to step (i) not contacted with ethanol may show the specific effect of the ethanol on the cell or non-human animal.

For comparative purposes, the following polypeptides are also mentioned herein:
5-hydroxytryptamine (serotonin) receptor 3 from the species origin Homo sapiens (ALPHA1_5HT3_Human) having the sequence of SEQ ID NO:5:
acetylcholine receptor beta subunit isoform 2 from the species origin Homo sapiens (Beta2_Human) having the sequence of SEQ ID NO:6:
acetylcholine receptor delta subunit from the species origin Torpedo *californica* (DELTA_TorpedoC) having the sequence of SEQ ID NO:7:
GABA-A receptor alpha 1 subunit from the species origin Homo sapiens (ALPHA1_GABA_A_Human) having the sequence of SEQ ID NO:8:
acetylcholine receptor gamma subunit from the species origin Torpedo *californica* (GAMMA_TorpedoC) having the sequence of SEQ ID NO:9:
acetylcholine receptor beta subunit isoform 1 from the species origin Torpedo californica (Beta1_TorpedoC) having the sequence of SEQ ID NO:10:
acetylcholine receptor alpha subunit isoform 1 from the species origin Homo sapiens (ALPHA7_Human) having the sequence of SEQ ID NO:11:
glycine receptor subunit alpha-1 from the species origin Homo sapiens (ALPHA1_Gly_Human) having the sequence of SEQ ID NO:12:
GABA-A receptor alpha 4 subunit from the species origin Homo sapiens (ALPHA4_GABA_A_Human) having the sequence of SEQ ID NO:13:
GABA-A receptor beta 1 subunit from the species origin Homo sapiens (BETA1_GABA_A_Human) having the sequence of SEQ ID NO:14:

The following examples are intended to illustrate the invention but not to limit the scope of protection conferred by the claims.

### Examples

### Example 1

### Impact of the Mutation on the Volume of the Ion Pore

### Method

The method according to Example 1 was based on atom molecular dynamics simulations and docking methods used for investigating Cys-loop receptors generally known in the art (Wang et al., 2008; Wang and Sine, 2010) to characterize the ethanol-induced alterations in the conformation of the membrane-embedded prototype nAChR comprising nAChR α-polypeptide(s) and to analyze its correlation with the potentiating effects of alcohol on the function of this receptor type.

Herein, the cryo-EM structure of the neuromuscular nicotinic acetylcholine receptor (National Center for Biotechnology Information (NCBI), Bethesda MD, USA, PubMed accession number PDB: 2BG9) was embedded in a fully solvated lipid bilayer palmitoyl-oleoylphosphatidylcholine (POPC) membrane (200 A x 200 A) and lipids within 0.8 A of the receptor protein were removed. The unified membrane-protein complex was subsequently solvated in TIP3P water and sodium and chloride ions were added to neutralize the net charge of the system. Under basal conditions (no ethanol or acetylcholine), the resulting system contained 696,630 atoms, including 174,874 TIP3P water molecules and 65 ions. For the molecular dynamics simulations, the highly scalable parallel simulation code NAMD 2.9 (Phillips et al., 2005) and the CHARMM27 force field (MacKerell et al., 1998) were used.

Herein, as the potential energy of a macromolecular system is a high-dimensional complex hypersurface, robust energy minimizations was used when the starting configuration of the system was far from equilibrium. Furthermore, the knowledge of all local minima of the potential energy allowed the investigation of all relevant structures and conformations, their free energies as well as the dynamics of structural transitions. However, the large number of local minima as well as the high dimension of the configuration space exacerbated a complete survey and suggested to focus the nearest local minimum, i.e. the achieved minimum by systematically moving down the steepest local gradient.

In the present study, a conjugate gradient method for 15000 time-steps (30 ps) was applied leading to a high accuracy level regarding the localization of minima. This method used the partial derivatives and function evaluations of the potential energy with respect to all known coordinates and possesses good convergence properties in near minima cases. In order to prevent the system from collapses due to unrestrained dynamical processes, the equilibration of the ensemble was performed on the "isothermal-isochoric" or "canonical" ensemble, assuming constant number of particles, volume, and temperature (NVT). Here, a 2 ns NVT equilibration was conducted, which is sufficient for the temperature of the system should reach the stable plateau at 310 °K due to the phase transition temperature of the POPC lipids. For all simulations the non-bonded interactions had a short cutoff of 9 A and periodic boundary conditions with Particle Mesh Ewald method were used. Langevin dynamics and a Langevin piston algorithm were used to control the temperature and pressure of the system and maintain the ensemble at 310 °K and a pressure of 1 atm. All simulations were performed on the computer cluster "Elwetritsch" of the University of Kaiserslautern (512 nodes with 16 processors per node) to simulate at least 100 ns in real-time. The simulation studies were performed in three steps. In the first stage, the solvated membrane-protein complex was simulated in order to demonstrate the robustness of the inactive closed receptor state without any pharmacological agent.

In addition, three simulation rounds were conducted to characterize the effects of 20 mM ethanol and 0.2 mM acetylcholine, alone and in combination, on the conformation of the nicotinic receptor.

Since a direct impact of ethanol on receptor structure and energy levels was found in the first stage, the second stage utilized Autodock Vina (Trott and Olson, 2010) to identify the most likely allosteric binding sites of alcohol by computational docking methods. Using a similar strategy as previous investigations (Sine et al., 2002), amino acid scanning mutagenesis was combined with molecular dynamics simulations in the third stage in order to characterize the nature of ethanol interactions with the extracellular moiety. Using *in silico* site directed mutagenesis, the several amino acid moieties were substituted by serine, glutamic acid, lysine and glycine in order to analyze the impact of charge, polarity and size properties of the moieties on the ethanol interactions. In this context, the entire docking cavity was substituted to mimic the respective cavities in nAChR α-polypeptides. Herein, the polypeptide sequence of the docking cavity of the wild type polypeptide included a sequence motif of SEQ ID NO:23.

In addition, a cavity-close moiety (the amino acid moiety located C-terminally of the sequence motif of SEQ ID NO:23) was mutated to investigate the spatial accuracy of the identified amino acid Xaa1 of the polypeptide motif of the sequence of Pro-Ser-Xaa1-Xaa2-Xaa3-Trp (SEQ ID NO:19) (i.e., Asp107 in SEQ ID NOs: 1 and 2 and Glu128 in SEQ ID NO:3) as an amino acid moiety of particular impact for ethanol-induced conformational changes.

In order to quantitatively analyze the observed effects, the software 3Vee (Voss and Gerstein, 2010) was utilized to calculate the pore-volume of the different conformations of the receptor in wild type and mutant states.

### Results

The highest docking score was allocated to the negatively charged extracellular amino acid moiety in position Xaa1 of the polypeptide motif of the sequence of Pro-Ser-Xaa1-Xaa2-Xaa3-Trp (SEQ ID NO:23) comprised in the nAChR α-polypeptide (i.e., Asp107 in SEQ ID NOs: 1 and 2 and Glu128 in SEQ ID NO:3). Simulations of mutant receptors wherein the amino acid moiety at said position was substituted revealed that substitutions of this moiety with non-negative amino acids is able to abolish the ethanol-induced conformational changes suggesting the importance of the negative charge of the moiety for the effects of alcohol.

It could be demonstrated that upon contacting *Torpedo* nAChR with ethanol, the volume of the ion pore of the nAChR comprising the wild type form of *Torpedo* nAChR α-polypeptide significantly increases, whereas, in contrast, it is even reduced in the mutated form wherein ASP107 is substituted.

| Receptor/Ligand | Receptor Volume (Ang³) | Volume % Closed State | Volume % Ethanol-Induced State |
|---|---|---|---|
| nAChR/H₂O | 54693 | 100% | |
| nAChR/ACh | 99262 | 181% | |
| nAChR/EtOH | 73506 | 134% | 100% |
| nAChR/ACh+EtOH | 100018 | 183% | 136% |
| nAChR(ASP107->SER107)/EtOH | 50773* | 93% | 69% |

| | | | |
|---|---|---|---|
| Acetylcholine (ACh) concentration: 0.2 mM Ethanol (EtOH) concentration: 20mM | | | |

In detail, direct comparison of the molecular dynamics simulations showed that ethanol induces a semi-open, intermediate state of the ion channel, thus, a kinetic state of the nAChRs comprising the wild type nAChR α-polypeptide that has been previously associated with partial agonism of this receptor family.

This process correlates directly with the ethanol- and acetylcholine-induced changes in the total energy of the receptor with respect to its inactive state showing that ethanol potentiates the activation process of the receptor by varying the total energy such that less energy is required to achieve the open state through acetylcholine binding.

Due to the high homology of nAChR α-polypeptides of different subtypes and of various species, these findings plausibly demonstrate that the present invention applies to nAChR α-polypeptides in general.

### References

MacKerell AD, Bashford D, Bellott, Dunbrack RL, Evanseck JD, Field MJ, Fischer S, Gao J, Guo H, Ha S, Joseph-McCarthy D, Kuchnir L, Kuczera K, Lau FTK, Mattos C, Michnick S, Ngo T, Nguyen DT, Prodhom B, Reiher WE, Roux B, Schlenkrich M, Smith JC, Stote R, Straub J, Watanabe M, Wiorkiewicz-Kuczera J, Yin D, Karplus M (1998) All-Atom Empirical Potential for Molecular Modeling and Dynamics Studies of Proteins. The Journal of Physical Chemistry B 102:3586-3616.
Phillips JC, Braun R, Wang W, Gumbart J, Tajkhorshid E, Villa E, Chipot C, Skeel RD, Kale L, Schulten K (2005) Scalable molecular dynamics with NAMD. J Comput Chem 26:1781-1802.
Sine SM, Wang HL, Bren N (2002) Lysine scanning mutagenesis delineates structural model of the nicotinic receptor ligand binding domain. J Biol Chem 277:29210-29223.
Trott O, Olson AJ (2010) AutoDock Vina: improving the speed and accuracy of docking with a new scoring function, efficient optimization, and multithreading. J Comput Chem 31:455-461.
Voss NR, Gerstein M (2010) 3V: cavity, channel and cleft volume calculator and extractor. Nucleic Acids Res 38:W555-562.
Wang HL, Cheng X, Taylor P, McCammon JA, Sine SM (2008) Control of cation permeation through the nicotinic receptor channel. PLoS Comput Biol 4:e41.
Wang HL, Sine SM (2010) Function of the Acetylcholine Receptor at the Atomic Scale. J Comput Theor Nanosci 7:2538-2542.

### Example 2

### Impact of the Mutation on the Membrane Potential

Although the outcome of Example 1 as laid out above, already provides an appropriate and robust demonstration of the technical effect achieved by polypeptides according to the present invention, molecular biology and electrophysiological methods are further applied to validate these results even further.

Recordings of whole-cell currents are used as valid measures for the effects of ethanol on wild type and mutated forms of nAChRs. Therefore, the ethanol-induced whole-cell currents of theses nAChRs are compared to show the mutation-induced ethanol-insensitivity in terms of lacking ethanol-induced potentiation in the respective whole-cell currents.

### Experimental Procedure

Human Embryonic Kidney 293 (HEK-293) cells are plated on 12-mm glass coverslips (Assistent, Germany) at a density of 1-2 x10⁴ cells/coverslip in Minimum Essential Medium (Invitrogen), supplemented with 10% fetal calf serum (Invitrogen), 1 mM glutamine and 1 mM penicillin/streptomycin. Two days after plating, the cells are co-transfected using a standard Ca²⁺-phosphate precipitation protocol with synthetized cDNAs (625 ng/coverslip) encoding full-length isomers of alphal, beta1, epsilon and delta subunits of the human nAChR.

Electrophysiological recordings are subsequently performed at room temperature (about 23 °C) approximately 48 h after transfection on cells showing GFP fluorescence. Transfected HEK-293 cells are lifted from the coverslip, and 0.2 mM acetylcholine is then fast-applied (piezo-driven system) in the presence and absence of 20 mM ethanol for 20 or 100 ms as indicated. Solution exchange time, measured with an open patch pipette, set around 100-200 µs. Extracellular solution contains (in mM): 135 NaCl, 5.4 KCI, 1.8 CaCl2, 5 HEPES, i.e., lacking a zinc chelator, or 0.01 mM ethylenediaminetetraacetic acid (EDTA) are added as indicated (pH 7.25).

Using these settings, whole-cell currents are recorded at a holding potential of -60 mV in solution containing 0.2 mMCaCl2. Patch pipettes (4-6 MΩ) are filled with (in mM): 140 CsCl, 2 MgATP, 10 EGTA, 10 HEPES (pH 7.25, 290-305 mOsm). The current traces are filtered at 3 kHz and digitized at 10 kHz using the AD-converter of the EPC-9 (HEKA, Germany), which was controlled by Pulse or Patchmaster software running on a Macintosh Power PC 9500/200.

### Obtainable results

These data then demonstrate that the cells bearing an nAChR comprising a mutated form of the nAChR α-polypeptide, wherein ASP83 is substituted, are less sensitive to ethanol compared to cells bearing a respective nAChR comprising a wild type form of the nAChR α-polypeptide only. The membrane potential alterations are less prominent in the mutated form when the cells are contacted with ethanol. Likewise, the Na⁺/K⁺ alterations are different and less prominent in the cells bearing an nAChR comprising a mutated form of the nAChR α-polypeptide. Therefore, also the signal transduction dependent on Na⁺/K⁺ alterations and/or membrane potential alterations is also different.

## Claims

1. A nicotinic acetylcholine receptor subunit alpha polypeptide comprising a polypeptide motif of the sequence of
Pro-Ser-Xaa1-Xaa2-Xaa3-Trp (SEQ ID NO:19)
wherein:
Xaa1 refers to Ser;
Xaa2 refers to Lys or Leu; and
Xaa3 refers to Ile.

2. The polypeptide according to claim 1, wherein the amino acid moiety Xaa1 is located at an amino acid moiety position between 100 and 150 in the amino acid of the nicotinic acetylcholine receptor subunit alpha polypeptide, in particular in a position of between 105 and 130 in the amino acid of the nicotinic acetylcholine receptor subunit alpha polypeptide.

3. The polypeptide according to any one of claims 1 to 3, wherein said polypeptide is selected from polypeptides having a sequence homology of at least 80% to any one of the sequences of SEQ ID NO:17, SEQ ID NO:18, SEQ ID NO:15 or SEQ ID NO:16.

4. A polynucleotide encoding for a polypeptide according to any one of claims 1 to 3, in particular wherein said polynucleotide is comprised in a vector.

5. A cell comprising a polypeptide according to any one of claims 1 to 3 and/or a polynucleotide according to claim 4, wherein said cell is not a human embryonic stem cell.

6. The cell according to claim 5, wherein said cell is essentially free of the wild type nicotinic acetylcholine receptor polypeptide corresponding to the polypeptide of any one of claims 1 to 3 present in the cells.

7. An non-human animal comprising cells according to claim 5 or 6, preferably wherein in at least one tissue essentially all cells of at least one cell type are cells according to claim 5 or 6, in particular wherein in at least one tissue essentially all cells of at least one cell type are cells according to claim 6.

8. The use of a polypeptide according to any one of claims 1 to 3, a nicotinic acetylcholine receptor comprising a polypeptide according to any one of claims 1 to 3, a polynucleotide according to claim 4, a cell according to claim 5 or 6, for determining the effect of a compound, in particular a drug compound, or a combination of compounds, on the activity of a nicotinic acetylcholine receptor comprising said polypeptide.

9. The use according to claim 8, wherein the activity of the nicotinic acetylcholine receptor is the rate of ions passing the ion channel of said receptor, in particular wherein the activity of the nicotinic acetylcholine receptor is determined in the absence of ethanol.

10. An *in vitro* method for determining the effect of at least one compound, in particular a drug compound, on the activity of a nicotinic acetylcholine receptor, comprising the polypeptide of any one of claims 1 to 3, said method comprising the steps:
(i) providing a polypeptide according to any one of claims 1 to 3 comprised in a nicotinic acetylcholine receptor in an environment maintaining its functionality;
(ii) contacting said nicotinic acetylcholine receptor comprising said polypeptide of step (i) with said at least one compound in an *in vitro* environment maintaining functionality of said polypeptide;
(iii) determining the activity of said acetylcholine receptor comprising said polypeptide contacted with said at least one compound of step (ii); and
(iv) determining the effect of said at least one compound by means of comparing the activity determined according to step (iii) with the activity determined for the acetylcholine receptor comprising said polypeptide of in a comparable *in vitro* environment, without being contacted with said at least one compound.

11. The method according to claim 10, wherein the environment maintaining the functionality of the acetylcholine receptor comprising said polypeptide is a membrane, composed of amphiphilic molecules, preferably a cellular membrane, more preferably the cellular membrane of a viable cell according to claim 5 or 6.

12. The method according to claim 10 or 11, wherein the step of determining the activity of the nicotinic acetylcholine receptor comprising said polypeptide (step iii) is determining the rate of ions passing the ion channel of said receptor.

13. The method according to any one of claims 10 to 12, wherein the method is conducted in the absence of ethanol.

14. An *in vitro* method for determining the effect of ethanol on a cell mediated by one or more nicotinic acetylcholine receptor(s) comprising one or more nicotinic acetylcholine receptor subunit alpha polypeptide(s) of interest, comprising the steps:
(i) providing a cell according to claim 6;
(ii) contacting said cell with ethanol;
(iii) determining the effect of the ethanol on said cell according to step (ii); and
(iv) comparing the effect of the ethanol on said cell according to step (iii) with
(a) a cell, only comprising the respective wild type nicotinic acetylcholine receptor(s); and/or
(b) a cell according to step (i), not contacted with ethanol.

## Patentansprüche

1. Polypeptid einer nikotinischen Acetylcholinrezeptor-Untereinheit Alpha umfassend ein Polypeptidmotiv der Sequenz
Pro-Ser-Xaa1-Xaa2-Xaa3-Trp (SEQ ID NO:19)
worin:
Xaa1 Ser bedeutet;
Xaa2 Lys oder Leu bedeutet; und
Xaa3 Ile bedeutet.

2. Das Polypeptid gemäß Anspruch 1, wobei der Aminosäurerest Xaa1 in einer Aminosäurerestposition zwischen 100 und 150 an Aminosäure des Polypeptids der nikotinischen Acetylcholinrezeptor-Untereinheit Alpha, bevorzugt in einer Position zwischen 105 und 130 an Aminosäure des Polypeptids der nikotinischen Acetylcholinrezeptor-Untereinheit Alpha, lokalisiert ist.

3. Polypeptid gemäß einem beliebigen der Ansprüche 1 bis 3, wobei das Polypeptid ausgewählt ist aus Polypeptiden, die eine Sequenzhomologie von mindestens 80% zu den Sequenzen SEQ ID NO:17, SEQ ID NO:18, SEQ ID NO:15 oder SEQ ID NO:16 aufweisen.

4. Polynukleotid, das für ein Polypeptid gemäß einem beliebigen der Ansprüche 1 bis 3 kodiert, insbesondere wobei das Polynukleotid von einem Vektor umfasst ist.

5. Zelle, die ein Polypeptid gemäß einem beliebigen der Ansprüche 1 bis 3 und/oder ein Polynukleotid gemäß Anspruch 4 umfasst, wobei die Zelle keine humane embryonale Stammzelle ist.

6. Zelle gemäß Anspruch 5, wobei die Zelle weitgehend frei von dem Wildtyp des Polypeptids des nikotinischen Acetylcholinrezeptors, das zu dem in den Zellen vorhandenen Polypeptid gemäß einem beliebigen der Ansprüche 1 bis 3 korrespondiert, ist.

7. Nichthumanes Tier, das Zellen gemäß Anspruch 5 oder 6 umfasst, wobei bevorzugt in mindestens einem Gewebe weitgehend alle Zellen mindestens eines Zelltyps Zellen gemäß Anspruch 5 oder 6 sind, wobei insbesondere in mindestens einem Gewebe weitgehend alle Zellen mindestens eines Zelltyps Zellen gemäß Anspruch 6 sind.

8. Verwendung eines Polypeptids gemäß einem beliebigen der Ansprüche 1 bis 3, eines nikotinischen Acetylcholinrezeptors umfassend ein Polypeptid gemäß einem beliebigen der Ansprüche 1 bis 3, eines Polynukleotids gemäß Anspruch 4, einer Zelle gemäß Anspruch 5 oder 6, zum Bestimmen der Wirkung einer Verbindung, insbesondere einer Arzneimittelverbindung, oder einer Kombination an Verbindungen auf die Aktivität eines nikotinischen Acetylcholinrezeptors umfassend das Polypeptid.

9. Verwendung gemäß Anspruch 8, wobei die Aktivität des nikotinischen Acetylcholinrezeptors die Rate an Ionen, die den Ionenkanal des Rezeptors passieren, ist, insbesondere wobei die Aktivität des nikotinischen Acetylcholinrezeptors in Abwesenheit von Ethanol bestimmt wird.

10. *In*-*vitro*-Verfahren zum Bestimmen der Wirkung mindestens einer Verbindung, insbesondere einer Arzneimittelverbindung, auf die Aktivität eines nikotinischen Acetylcholinrezeptors umfassend ein Polypeptid gemäß einem beliebigen der Ansprüche 1 bis 3, wobei das Verfahren die Schritte umfasst:
(i) Bereitstellen eines Polypeptids gemäß einem beliebigen der Ansprüche 1 bis 3, das von einem nikotinischen Acetylcholinrezeptor umfasst ist, in einer Umgebung, die dessen Funktionalität aufrecht erhält;
(ii) Inkontaktbringen des nikotinischen Acetylcholinrezeptors des Schritts (i) mit der mindestens einen Verbindung in einer *In*-*vitro*-Umgebung, die die Funktionalität des Polypeptids aufrecht erhält;
(iii) Bestimmen der Aktivität des mit der mindestens einen Verbindung nach Schritt (ii) in Kontakt gebrachten Acetylcholinrezeptors umfassend das Polypeptid; und
(iv) Bestimmen der Wirkung der mindestens einen Verbindung mittels Vergleichen der gemäß Schritt (iii) bestimmten Aktivität mit der Aktivität, die für den Acetylcholinrezeptor umfassend das Polypeptid in einer vergleichbaren *In*-*vitro*-Umgebung bestimmt wurde ohne mit der mindestens einen Verbindung in Kontakt gebracht worden zu sein.

11. Verfahren gemäß Anspruch 10, wobei die Umgebung, die die Funktionalität des Acetylcholinrezeptors umfassend das Polypeptid aufrecht erhält, eine Membran ist, die aus amphiphilen Molekülen aufgebaut ist, bevorzugt eine zelluläre Membran, stärker bevorzugt eine zelluläre Membran einer lebensfähigen Zelle gemäß Anspruch 5 oder 6, ist.

12. Verfahren gemäß Anspruch 10 oder 11, wobei der Schritt des Bestimmens der Aktivität des nikotinischen Acetylcholinrezeptors umfassend das Polypeptid (Schritt iii) das Bestimmen der Rate an Ionen, die den Ionenkanal des Rezeptors passieren, ist.

13. Verfahren gemäß einem beliebigen der Ansprüche 10 bis 12, wobei das Verfahren in der Abwesenheit von Ethanol durchgeführt wird.

14. *In*-*vitro*-Verfahren zum Bestimmen der Wirkung von Ethanol auf eine Zelle, die durch einen oder mehrere nikotinische(n) Acetylcholinrezeptor(en) umfassend ein oder mehrere Polypeptid(e) von nikotinischer Acetylcholinrezeptor-Untereinheit Alpha von Interesse, vermittelt wird, umfassend die Schritte:
(i) Bereitstellen einer Zelle gemäß Anspruch 6;
(ii) Inkontaktbringen der Zelle mit Ethanol;
(iii) Bestimmmen der Wirkung des Ethanols auf die Zelle gemäß Schritt (ii); und
(iv) Vergleichen der Wirkung des Ethanols auf die Zelle gemäß Schritt (iii) mit
(a) einer Zelle, die nur entsprechenden Wildtyp an nikotinischem Acetylcholinrezeptor bzw. nikotinischen Acetylcholinrezeptoren umfasst; und/oder
(b) einer nicht mit Ethanol in Kontakt gebrachten Zelle gemäß Schritt (i).

## Revendications

1. Polypeptide de sous-unité alpha de récepteur nicotinique d'acétylcholine comprenant un motif polypeptidique de la séquence de
Pro-Ser-Xaa1-Xaa2-Xaa3-Trp (SEQ ID NO: 19)
dans lequel :
Xaa1 désigne Ser ;
Xaa2 désigne Lys ou Leu ; et
Xaa3 désigne Ile.

2. Polypeptide selon la revendication 1, dans lequel le fragment d'acides aminés Xaa1 est situé à une position de fragment d'acides aminés entre 100 et 150 dans les acides aminés du polypeptide de la sous-unité alpha de récepteur nicotinique d'acétylcholine, en particulier à une position entre 105 et 130 dans les acides aminés du polypeptide de la sous-unité alpha de récepteur nicotinique d'acétylcholine.

3. Polypeptide selon l'une quelconque des revendications 1 à 3, ledit polypeptide étant choisi parmi des polypeptides ayant une homologie de séquence d'au moins 80 % avec l'une quelconque des séquences de SEQ ID NO: 17, SEQ ID NO: 18, SEQ ID NO: 15 ou SEQ ID NO: 16.

4. Polynucléotide codant pour un polypeptide selon l'une quelconque des revendications 1 à 3, ledit polynucléotide étant, en particulier, compris dans un vecteur.

5. Cellule comprenant un polypeptide selon l'une quelconque des revendications 1 à 3 et/ou un polynucléotide selon la revendication 4, ladite cellule n'étant pas une cellule souche embryonnaire humaine.

6. Cellule selon la revendication 5, ladite cellule étant pratiquement exempte de polypeptide de récepteur nicotinique d'acétylcholine de type sauvage correspondant au polypeptide de l'une quelconque des revendications 1 à 3 présent dans les cellules.

7. Animal non humain comprenant des cellules selon la revendication 5 ou 6, de préférence dans lequel, dans au moins un tissu, pratiquement toutes les cellules d'au moins un type de cellule sont des cellules selon la revendication 5 ou 6, en particulier dans lequel, dans au moins un tissu, pratiquement toutes les cellules d'au moins un type de cellule sont des cellules selon la revendication 6.

8. Utilisation d'un polypeptide selon l'une quelconque des revendications 1 à 3, un récepteur nicotinique d'acétylcholine comprenant un polypeptide selon l'une quelconque des revendications 1 à 3, un polynucléotide selon la revendication 4, une cellule selon la revendication 5 ou 6, pour déterminer l'effet d'un composé, en particulier un composé pharmaceutique, ou une combinaison de composés, sur l'activité d'un récepteur nicotinique d'acétylcholine comprenant ledit polypeptide.

9. Utilisation selon la revendication 8, dans laquelle l'activité du récepteur nicotinique d'acétylcholine est le taux d'ions traversant le canal ionique dudit récepteur, en particulier dans laquelle l'activité du récepteur nicotinique d'acétylcholine est déterminée en l'absence d'éthanol.

10. Procédé *in vitro* de détermination de l'effet d'au moins un composé, en particulier un composé pharmaceutique, sur l'activité d'un récepteur nicotinique d'acétylcholine, comprenant le polypeptide de l'une quelconque des revendications 1 à 3, ledit procédé comprenant les étapes de :
(i) fourniture d'un polypeptide selon l'une quelconque des revendications 1 à 3 compris dans un récepteur nicotinique d'acétylcholine dans un environnement maintenant sa fonctionnalité ;
(ii) mise en contact dudit récepteur nicotinique d'acétylcholine comprenant ledit polypeptide de l'étape (i) avec ledit au moins un composé dans un environnement *in vitro* maintenant la fonctionnalité dudit polypeptide ;
(iii) détermination de l'activité dudit récepteur d'acétylcholine comprenant ledit polypeptide mis en contact avec ledit au moins un composé de l'étape (ii) ; et
(iv) détermination de l'effet dudit au moins un composé au moyen de la comparaison de l'activité déterminée selon l'étape (iii) à l'activité déterminée pour le récepteur d'acétylcholine comprenant ledit polypeptide dans un environnement *in vitro* comparable, sans être mis en contact avec ledit au moins un composé.

11. Procédé selon la revendication 10, dans lequel l'environnement maintenant la fonctionnalité du récepteur d'acétylcholine comprenant ledit polypeptide est une membrane, composée de molécules amphiphiles, de préférence une membrane cellulaire, plus préférablement la membrane cellulaire d'une cellule viable selon la revendication 5 ou 6.

12. Procédé selon la revendication 10 ou 11, dans lequel l'étape de détermination de l'activité du récepteur nicotinique d'acétylcholine comprenant ledit polypeptide (étape iii) est la détermination du taux d'ions traversant le canal ionique dudit récepteur.

13. Procédé selon l'une quelconque des revendications 10 à 12, le procédé étant conduit en l'absence d'éthanol.

14. Procédé *in vitro* de détermination de l'effet de l'éthanol sur une cellule médié par un ou plusieurs récepteur(s) nicotinique(s) d'acétylcholine comprenant un ou plusieurs polypeptide(s) de sous-unité alpha de récepteur nicotinique d'acétylcholine d'intérêt, comprenant les étapes de :
(i) fourniture d'une cellule selon la revendication 6 ;
(ii) mise en contact de ladite cellule avec de l'éthanol ;
(iii) détermination de l'effet de l'éthanol sur ladite cellule selon l'étape (ii) ; et
(iv) comparaison de l'effet de l'éthanol sur ladite cellule selon l'étape (iii) à
(a) une cellule, comprenant uniquement le(s) récepteur(s) nicotinique(s) d'acétylcholine de type sauvage ; et/ou
(b) une cellule selon l'étape (i), non mise en contact avec de l'éthanol.
